# EUROPEAN PATENT APPLICATION

(11) **EP 1 103 603 A2**
(43) Date of publication of application: **30.05.2001**
(21) Application number: 00124785.7
(22) Date of filing: 14.11.2000
(51) Int. Cl.: C12N 9/02, C12N 15/53, C12N 1/21, C12P 7/60

(54) **Cytochrome c oxidase complex from Gluconobacter oxydans**

(30) Priority: 17.11.1999 EP 99122842
(71) Applicant: F. HOFFMANN-LA ROCHE AG, 4070 Basel (CH)
(72) Inventor: Asakura, Akira, Fujisawa-shi, Kanagawa-ken 251-0032 (JP); Hoshino, Tatsuo, Kamakura-shi, Kanagawa-ken 248-0027 (JP); Shinjoh, Masako, Kamakura-shi, Kanagawa-ken 247-0061 (JP)
(74) Representative: Kellenberger, Marcus Dr.

(57) **Abstract**

The present invention is directed to a novel cytochrome c oxidase complex, genetic materials useful for the preparation of the said complex, such as recombinant polypeptides involved in cytochrome c oxidase complex, recombinant DNA fragments, expression vectors, recombinant organisms and the like. Those novel cytochrome c oxidase complex and genetic materials may be originated from a microorganisn having the identifying characteristics of Gluconobacter oxydans DSM 4025. The present invention also provides a method for the preparation of the said novel recombinant cytochrome c oxidase complex and a process for the production of 2-keto-L-gulonic acid (2KGA).

## Description

The present invention relates to recombinant production of 2-keto-L-gulonic acid and biological materials useful thereof.

Cytochrome c oxidase (cytochrome aa3; EC 1.9.3.1) is a terminal oxidase enzyme in the aerobic respiratory electron transport system of mitochondria and many bacteria. The enzyme is a cytoplasmic membrane spanning complex and catalyzes the final electron excreting; re-oxidation of ferrocytochrome c (electron donor) at periplasmic surface / reduction of molecular oxygen (electron acceptor) to water at cytoplasmic surface. The reaction couples with extrusion of protons across the membrane and this coupling is indispensable for biological energy conservation from substrate oxidation.

Various types of cytochrome complex, e.g. aa3, al, caa3, o, bo, co, bd-types, have been identified as functional terminal oxidases; purification and characterization of some terminal oxidases have been reported. Matsushita et al. reported that Acetobacter aceti IFO 3283 contains two terminal oxidases, cytochrome al and o, and purified and characterized the cytochrome al (Proc. Natl. Acad. Sci., USA, 87: 9863, 1990; J. Bacteriol. 174: 122, 1992). Matsushita et al. also reported the purification of cytochrome o from Gluconobacter (Biochem. Biophys. Acta, 894: 304, 1987). Tayama et al. disclosed a terminal oxidase (cytochrome al) genes of A. aceti (JP 93-317054); they purified the oxidase enzyme which consisted of four subunits of 72, 34, 21, and 13 kDa and contained heme a and heme b. The oxidases in Acetobacter and Gluconobacter belong to quinol oxidase. Cytochrome aa3 (cytochrome c oxidase) has been purified from bovine heart, yeast, and many bacteria including Paracoccous denitrificans (Solioz et al., J. Biol. Chem., 257: 1579-1582, 1982) and Rhodobacter sphaeroides (Hosler et al., J. Biol. Chem., 267: 24264-24272, 1992).

Mammalian (mitochondorial) cytochrome c oxidase (aa3-type) complex contains 13 different subunits; the three core subunits I, II and III (CO I, II and III) are encoded by the mitochondorial DNA, while the remaining 10 are by nuclear origin. Bacterial aa3-type cytochrome c oxidase also contains the three core subunits, which are homologous to the mitochondorial core subunits. However, it is reported that CO III was easily lost during purification, resulting in preparations composed of CO I and CO II only (Ludwig et al., Proc. Natl. Acad. Sci. USA, 77: 196-200, 1980). The cytochrome c oxidase complex consisting of the two-subunit (CO I and II) showed redox activity with generation of electrochemical proton gradient. In case of P. denitrificans (Haltia et al., The EMBO Journal, 10: 2015-2021, 1991) and R. sphaeroides (Cao et al., Gene, 101: 133-137, 1991), both two-subunit-type (CO I / II) and three-subunit-type (CO I / II / III) of complexes were isolated by different purification methods. Genetically, genes for CO II and III are located in an operon, while gene for CO I is independently located (Raitio et al., The EMBO Journal, 9: 2825-2833, 1987, Shapleigh et al., Proc. Natl. Acad. Sci. USA, 89: 4786-4790, 1992).

Terminal oxidases as described above play an important role in growth under aerobic condition to accomplish reduction of molecular oxygen. In oxidative fermentation, respiratory chain including terminal oxidase works for completing oxidation of a substrate to produce an oxidized product. In this context, it is very important to improve the efficiency of the respiratory chain for efficient oxidative fermentation.

G. oxydans DSM 4025 produces 2-keto-L-gulonic acid (hereinafter: 2KGA), an important intermediate in a process of L-ascorbic acid production, from L-sorbose via L-sorbosone (T. Hoshino et al., EP 0 366 922 A). The oxidation of the substrate, L-sorbose, to 2KGA was considered to be completed by the respiratory electron transport chain. The terminal oxidase which catalyzes the final electron excreting to oxygen might be one of the kinetic rate-limiting steps in the 2KGA production system as well as other redox components. The primary dehydrogenase responsible for 2KGA formation from L-sorbose was isolated (T. Hoshino et al., EP 606621 A) and the genes were cloned and sequenced; four isozymes were found (T. Hoshino et al., EP 832974 A) and their direct electron acceptor, cytochrome c551, was purified and its gene was cloned (T. Hoshino et al., EP 0869175 A). The terminal oxidase was, however, not isolated and its genes were not cloned.

The present invention was aimed for providing the materials for improving the quantity and quality of cytochrome c oxidase and improving oxidative fermentation completed by the cytochrome c oxidase with the aid of making novel cytochrome c oxidase genes available. The microorganism deposited as Gluconobacter oxydans under the accession No. DSM 4025 is a preferable example of a source which provides the novel cytochrome c oxidase and its genetic materials of the present invention.

The present invention provides a novel cytochrome c oxidase enzyme complex which are isolated from natural source or prepared with the aid of genetic engineering. Such an enzyme complex having cytochrome c oxidase activity are obtainable or obtained from biological or genetic material originated from a microorganism identified as G. oxydans DSM 4025 or biologically and/or taxonomically homogeneous cultures of a microorganism having the identifying characteristics of G. oxydans DSM 4025. Thus the present invention provides a novel cytochrome c oxidase complex which is useful as an essential component mediating electron transfer in the respiratory chain.

A cytochrome c oxidase complex exemplified herein is the one which shows the following physicochemical properties of: (i) showing the presence of at least two core subunits of I (COI) and II (COII) wherein; apparent molecular mass of COI is about 43 +/- 10 kDa by SDS-PAGE analysis; and apparent molecular mass of COII is about 36 +/-10 kDa by SDS-PAGE analysis, and (ii) absorption spectrum showing aa3-type cytochrome c oxidase being; 605 +/- 1 nm peak in reduced minus oxidized difference spectrum. Such a cytochrome c oxidase complex can be provided as a substantially homogeneous isolate derived from a culture of a microorganism identified as G. oxydans DSM 4025 or biologically and/or taxonomically homogeneous cultures of a microorganism having the identifying characteristics of G. oxydans DSM 4025.

A novel cytochrome c oxidase complex of the present invention can be also provided in the form of a recombinant enzyme, which may comprise a recombinant polypeptide as a core subunit I (COI), wherein the recombinant polypeptide can be selected from the group consisting of polypeptides having an amino acid sequence identified by SEQ ID NO: 2 and those having amino acid sequences having 85% or higher identity with the said sequence and being capable of providing the complex with cytochrome c oxidase activity. Further, the other core subunit II (COII) and III (COIII) can be recombinant polypeptide(s) selected from the group consisting of those containing amino acid sequences identified by SEQ ID NOs: 4, 6 and/or 8 and those containing amino acid sequences having 85% or higher identity with any one of the said amino acid sequences, and is (are) capable of providing the complex with cytochrome c oxidase activity.

As another aspect of the present invention, the respective core subunits, i.e. COI, COII and COIII are provided as recombinant polypeptides which are useful for the components of the novel cytochrome c oxidase complex of the present invention.

Exemplified herein as COI is a recombinant polypeptide which is involved in cytochrome c oxidase complex, which polypeptide comprises an amino acid sequence identified by SEQ ID NO: 2 or an amino acid sequence having 85 % or higher identity with the said amino acid sequence and is capable of providing the complex with cytochrome c oxidase activity. A recombinant COI may be a polypeptide capable of providing the complex of the present invention with cytochrome c oxidase activity, which is encoded by a recombinant DNA fragment comprising a DNA sequence selected from the group consisting of:
(a) the DNA sequence identified by SEQ ID NO: 1, and
(b) the DNA sequences which encode polypeptides having an amino acid sequence identified by SEQ ID NO: 2 or amino acid sequences having 85 % or higher identity with the said amino acid sequence.

Also exemplified herein as COII is a recombinant polypeptide which is involved in cytochrome c oxidase complex of the present invention, which polypeptide contains an amino acid sequence identified by SEQ ID NO: 4, or an amino acid sequence having 85 % or higher identity with the said amino acid sequence and is capable of providing the said complex with cytochrome c oxidase activity. A recombinant COII may be a polypeptide capable of providing the complex of the present invention with cytochrome c oxidase activity, which is encoded by a recombinant DNA fragment containing a DNA sequence selected from the group consisting of:
(a) the DNA sequence identified by SEQ ID NO: 3, and
(b) the DNA sequences which encode polypeptides having an amino acid sequence identified by SEQ ID NO: 4 or amino acid sequences having 85 % or higher identity with the said amino acid sequence.

Moreover, also exemplified herein as COIII is a recombinant polypeptide which is involved in cytochrome c oxidase complex of the present invention. Such recombinant polypeptide contains either or both of the amino acid sequences identified by SEQ ID NOs: 6 and 8, respectively or amino acid sequences having 85 % or higher identity with the respective amino acid sequences of SEQ ID NOs: 6 and 8 and is capable of providing the said complex with cytochrome c oxidase activity. A recombinant COIII may be a recombinant polypeptide capable of providing the complex of the present invention with cytochrome c oxidase activity, which is encoded by a recombinant DNA fragment containing one or more DNA sequence(s) selected from the group consisting of:
(a) the DNA sequence identified by SEQ ID NO: 5,
(b) the DNA sequence identified by SEQ ID NO: 7,
(c) the DNA sequences which encode polypeptides having an amino acid sequence identified by SEQ ID NO: 6 or amino acid sequences having 85 % or higher identity with the said amino acid sequence, and
(d) the DNA sequences which encode polypeptides having an amino acid sequence identified by SEQ ID NO: 8 or amino acid sequences having 85 % or higher identity with the said amino acid sequence.

In further aspect of the present invention, recombinant DNA fragments which are useful for preparing the respective core subunits, i.e. COI, COII and COIII by genetic engineering are provided. Such recombinant polypeptides are useful for the components to be involved in the novel cytochrome c oxidase complex of the present invention. As explained above, these polypeptides should be capable of providing the cytochrome c oxidase complex of the present invention with cytochrome c oxidase activity

Exemplified herein as a recombinant DNA fragment for COI is a DNA fragment which encodes a polypeptide involved in cytochrome c oxidase complex and comprises a DNA sequence selected from the group consisting of:
(a) the DNA sequence identified by SEQ ID NO: 1, and
(b) the DNA sequences which encode polypeptides having an amino acid sequence identified by SEQ ID NO: 2 or amino acid sequences having 85 % or higher identity with the said amino acid sequence.

Also exemplified herein as a recombinant DNA fragment for COII is a DNA fragment which encodes a polypeptide involved in cytochrome c oxidase complex and contains a DNA sequence selected from the group consisting of:
(a) the DNA sequence identified by SEQ ID NO: 3, and
(b) the DNA sequences which encode polypeptides having an amino acid sequence identified by SEQ ID NO: 4 or amino acid sequences having 85 % or higher identity with the said amino acid sequence.

Moreover, also exemplified herein as a recombinant DNA fragment for COIII is a DNA fragment which encodes a polypeptide involved in cytochrome c oxidase complex and contains one or more DNA sequence(s) selected from the group consisting of:
(a) the DNA sequence identified by SEQ ID NO: 5,
(b) the DNA sequence identified by SEQ ID NO: 7,
(c) the DNA sequences which encode polypeptides having an amino acid sequence identified by SEQ ID NO: 6 or amino acid sequences having 85 % or higher identity with the said amino acid sequence, and
(d) the DNA sequences which encode polypeptides having an amino acid sequence identified by SEQ ID NO: 8 or amino acid sequences having 85 % or higher identity with the said amino acid sequence.

Moreover, in another aspect of this invention, it is provided an expression vector comprising one or more of the above mentioned recombinant DNA fragments, which vector is suitable for the expression in an organism, pro- or eukaryotic host cell.

Further, it is another aspect of the present invention to provide a recombinant organism which is introduced with an expression vector mentioned above. Such a recombinant organism of the invention will be useful for the genetic preparation of a recombinant cytochrome c oxidase complex of the present invention and also applicable to a process for producing 2KGA from L-sorbose or D-sorbitol in an appropriate culture medium. Host cells for the recombinant organism of the present invention may be of eukaryotic origin, preferably a mammalian or plant cell, or may be of prokaryotic origin. These host cells may in particular be obtained from bacteria, preferably G. oxydans DSM 4025 and biologically and/or taxonomically homogeneous cultures of a microorganism having the identifying characteristics of Gluconobacter oxydans DSM 4025.

This invention is also directed to a process for producing cytochrome c oxidase, which comprises cultivating the recombinant organism of this invention mentioned above, particularly the recombinant organism containing a preferred DNA sequence exemplified herein, in an appropriate culture medium and recovering the cytochrome c oxidase from the culture medium.

Further, this invention is also directed to a process for producing 2KGA from L-sorbose or D-sorbitol which comprises cultivating a recombinant organism of the present invention as mentioned above in an appropriate culture medium and recovering 2KGA from the culture.

The following figures are included to further illustrate the present invention together with the detailed description given below.

Figure 1 shows absorption spectra pattern of aa3-type cytochrome c oxidase of G. oxydans DSM 4025. Spectra were recorded at room temperature at a protein concentration of 0.08 mg/ml in 25 mM Na-HEPES (pH 7.5) containing 0.5% sucrose monolaurate and 5% glycerol. [A] shows the spectrum of the oxidized form. [B] shows the spectrum of the reduced form. [C] shows reduced minus oxidized difference spectrum.

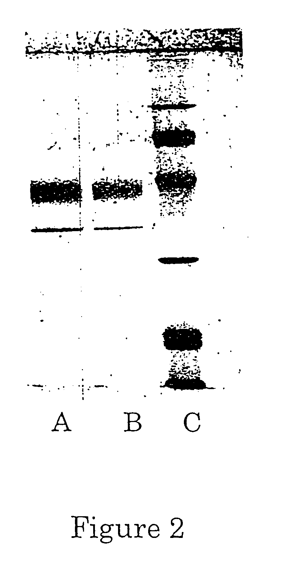

Figure 2 shows SDS-PAGE analysis of the purified cytochrome c oxidase aa3 of G. oxydans DSM 4025. The purified enzyme (at 0.5 mg/ml of protein concentration) was denatured by incubation with 2% SDS, 50 mM dithioerythritol, 62.5 mM Tris-HCl (pH 6.8) and 10% glycerol at 37oC for 5 hours. Electrophoresis was carried out at 12.5% acrylamide concentration according to the method of Laemmli (Nature, 227: 680-685, 1970) with the buffer consisting of 25 mM Tris, 0.192 M glycine, and 0.1% SDS. A and B were 6 and 3 microgram of the purified enzyme, respectively. C was low range prestained SDS-PAGE standards (Bio-Rad Laboratories, CA U.S.A.).

Figure 3 shows an alignment of the partial amino acid sequences of CO I from G. oxydans DSM 4025 with ones from other organisms.

Figure 4 shows an alignment of the partial amino acid sequences of CO II from G. oxydans DSM 4025 with ones from other organisms.

Figure 5 shows an alignment of the partial amino acid sequences of CO III from G. oxydans DSM 4025 with ones from other organisms.

Figure 6 shows primers for PCR amplification of the partial CO I, II and III genes of the cytochrome c oxidase complex from G. oxydans DSM 4025.

Figure 7 shows the physical maps of the 8.0 kb PstI and 9.3 kb EcoRI fragments containing "CO I" and "CO II and III" genes, respectively.

Figure 8 shows an alignment of the complete amino acid sequence of the CO I subunit from G. oxydans DSM 4025 with ones from other organisms.

Figure 9 shows a genetic map of pVKcoxes used for expressing the genes of cytochrome c oxidase complex of G. oxydans DSM 4025.

The novel cytochrome c oxidase complex of the present invention belongs to a family of proteins which function as a terminal oxidase and its genes. More particularly, the novel cytochrome c oxidase of the present invention is useful as a terminal oxidase oxidizing cytochrome c, an electron acceptor for dehydrogenases such as alcohol and aldehyde dehydrogenase (AADH) and thus is useful as essential component mediating electron transfer in the respiratory chain. A cytochrome c oxidase complex of the present invention may be those isolated from natural source or prepared with the aid of genetic engineering. Such an enzyme complex having cytochrome c oxidase activity is obtainable from biological material originated from a microorganism identified as G. oxydans DSM 4025 or biologically and/or taxonomically homogeneous cultures of a microorganism having the identifying characteristics of G. oxydans DSM 4025. The cytochrome c oxidase complex of the present invention shows the following physico-chemical characteristics: the complex shows an absorption of aa3-type cytochrome c oxidase in reduced minus oxidized difference spetrum, a peak at 605 +/- 1 nm; two polypeptides involved in the cytochrome c oxidase complex have apparent molecular masses of about 43 +/- 10 kDa and 36 +/- 10 kDa on SDS-PAGE.

As used herein, the phrase "a biologically and/or taxonomically homogeneous culture of a microorganism having the identifying characteristics of G. oxydans DSM 4025" means a microorganism that has at least 12 out of 14 of the following characteristics of G. oxydans DSM 4025:
(a) produces 2-KGA from L-sorbose,
(b) oxidizes ethanol to acetic acid,
(c) oxidizes D-glucose to D-gluconic acid and 2-keto-D-gluconic acid,
(d) exhibits ketogenesis of polyalcohols,
(e) exhibits pellicle and ring growth in mannitol broth (24 hour cultivation) at pH 4 and 5, and pellicle growth in glucose broth at pH 4.5,
(f) does not substantially oxidize glycerol to dihydroxyacetone,
(g) produces 2-keto-D-glucaric acid from sorbitol and glucaric acid but not from glucose, fructose, gluconic acid, mannitol or 2-keto-D-gluconic acid,
(h) is polymorphic, with no apparent flagella,
(i) produces brown pigment from fructose,
(j) exhibits good growth when co-cultured in the presence of B. megaterium or a cell extract thereof,
(k) is streptomycin sensitive,
(l) is rod-shaped with rounded ends,
(m) has an average cell diameter of about 0.3-0.6 micrometers,
(n) has an average cell length of about 1-1.5 micrometers; and
which microorganism produces 2-KGA from L-sorbose on the level of at least 0.01 g/L of 2-KGA in the culture medium as measured by HPLC. In addition to this, the phrase "a biologically and/or taxonomically homogeneous culture of a microorganism having the identifying characteristics of G. oxydans DSM 4025" should be understood to encompass a microorganism comprising a polynucleotide sequence which hybridizes under high stringency conditions to a polynucleotide sequence which encodes a polypeptide selected from the group consisting of SEQ ID NO:2, 4, 6, and 8, as it is obvious for the person skilled in the art that such a microorganism can be identified based on homology of the amino acid sequences.

A novel recombinant enzyme complex of the present invention can be prepared by using genetic material, i.e. recombinant DNA fragments originated from a microorganism identified as G. oxydans DSM 4025 or a biologically and/or taxonomically homogeneous cultures of a microorganism having the identifying characteristics of G. oxydans DSM 4025. Such a novel cytochrome c oxidase complex may comprise at least one recombinant polypeptide as one of the core subunits. The recombinant polypeptide as the core subunit I of the said complex can be selected from the group consisting of polypeptide having an amino acid sequence identified by SEQ ID NO: 2 and those having amino acid sequences having 85% or higher identity with the said sequence and being capable of providing the complex with cytochrome c oxidase activity. As well, either or both of the other core subunits II (COII) and III (COIII) can be recombinant polypeptide(s). COII may be selected from the group consisting of recombinant polypeptides containing partial amino acid sequence identified by SEQ ID NO: 4 and those containing partial amino acid sequence having 85% or higher identity with the said amino acid sequence, as long as such recombinant polypeptides are capable of providing the complex with cytochrome c oxidase activity. COIII may be selected from the group consisting of recombinant polypeptides containing partial amino acid sequences identified by SEQ ID NOs: 6 and 8 and those containing partial amino acid sequences having 85% or higher identity with the respective amino acid sequences, as long as such recombinant polypeptides are capable of providing the complex with cytochrome c oxidase activity.

The term "identity" has preferably the meaning that the amino acids occurring at the respective positions are not only similar with regard to their properties but are in fact identical. In a preferred embodiment the alignment of the amino acid sequences is performed in best mode.

The present invention is also directed to the polypeptides involved in the said cytochrome c oxidase complex. The polypeptides involved in the said cytochrome c oxidase complex and the amino acid sequences described in SEQ ID NOs: 2, 4, 6 and 8 showed homologies of 50-82 % at most with the polypeptides or the corresponding partial amino acid sequences involved in other cytochrome oxidases. For example, the CO I polypeptide of the present invention (SEQ ID NO: 2) showed 77%, 81% and 79% homologies with CO I alpha (accession No. P08305) and CO I beta (accession No. P98002) from P. denitrificans and CO I from R. sphaeroides (accession No. P33517), respectively. The partial CO II polypeptide of the present invention (SEQ ID NO: 4) showed 73% and 68% with the CO II polypeptides from P. denitrificans and R. sphaeroides, respectively. The partial CO III polypeptide of the present invention (SEQ ID NO: 6 ) showed 54% with the CO III polypeptide from P. denitrificans and another polypeptide (SEQ ID NO: 8) showed 71% and 63% with the CO III polypeptides from P. denitrificans and R. sphaeroides, respectively. These homology searches can be done by a computor programm such as "Search Homology" of Genetyx-SV/RC version 3.2.0 (Genetyx Software Development Co. Ltd., Tokyo Japan).

Thus the respective core subunits, i.e. COI, COII and COIII may be provided as recombinant polypeptides which are useful for the components of the novel cytochrome c oxidase complex of the present invention.

The subunit, COI of the complex may be a recombinant polypeptide which is involved in cytochrome c oxidase complex of the present invention, which polypeptide comprises an amino acid sequence identified by SEQ ID NO: 2 or an amino acid sequence having 85 % or higher identity with the said amino acid sequence and is capable of providing the complex with cytochrome c oxidase activity, as described above. A recombinant COI may also be a polypeptide capable of providing the complex of the present invention with cytochrome c oxidase activity, which is encoded by a recombinant DNA fragment comprising a DNA sequence selected from the group consisting of:
(a) the DNA sequence identified by SEQ ID NO: 1, and
(b) the DNA sequences which encode polypeptides having an amino acid sequence identified by SEQ ID NO: 2 or amino acid sequences having 85 % or higher identity with the said amino acid sequence.

Also the subunit, COII may be a recombinant polypeptide which is involved in cytochrome c oxidase complex of the present invention, which polypeptide contains an amino acid sequence identified by SEQ ID NO: 4, or an amino acid sequence having 85 % or higher identity with the said amino acid sequence and is capable of providing the said complex with cytochrome c oxidase activity, as described above. A recombinant COII may also be a polypeptide capable of providing the complex of the present invention with cytochrome c oxidase activity, which is encoded by a recombinant DNA fragment containing a DNA sequence selected from the group consisting of:
(a) the DNA sequence identified by SEQ ID NO: 3, and
(b) the DNA sequences which encode polypeptides having an amino acid sequence identified by SEQ ID NO: 4 or amino acid sequences having 85 % or higher identity with the said amino acid sequence.

Moreover, the subunit, COIII may be a recombinant polypeptide which is involved in cytochrome c oxidase complex of the present invention, which polypeptide contains either or both of the amino acid sequences identified by SEQ ID NOs: 6 and 8, respectively or amino acid sequences having 85 % or higher identity with the respective amino acid sequences of SEQ ID NOs: 6 and 8, as long as such recombinant polypeptides are capable of providing the said complex with cytochrome c oxidase activity, as described above. A recombinant COIII may also be a recombinant polypeptide capable of providing the complex of the present invention with cytochrome c oxidase activity, which is encoded by a recombinant DNA fragment containing one or more DNA sequence(s) selected from the group consisting of:
(a) the DNA sequence identified by SEQ ID NO: 5,
(b) the DNA sequence identified by SEQ ID NO: 7,
(c) the DNA sequences which encode polypeptides having an amino acid sequence identified by SEQ ID NO: 6 or amino acid sequences having 85 % or higher identity with the said amino acid sequence, and
(d) the DNA sequences which encode polypeptides having an amino acid sequence identified by SEQ ID NO: 8 or amino acid sequences having 85 % or higher identity with the said amino acid sequence.

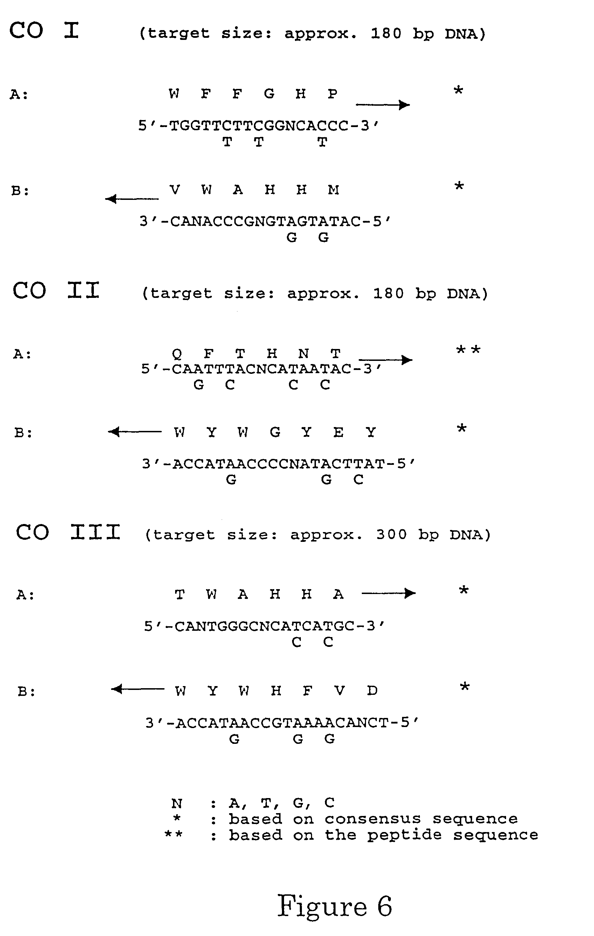

Furthermore the present invention encompasses functional derivatives of the recombinant polypeptides described above. Such functional derivatives are defined on the basis of the amino acid sequence of the present invention by addition, insertion, deletion and/or substitution of one or more amino acid residues of such sequences where cytochrome c oxidase complex comprising such derivatives still have cytochrome c oxidase activity measured by an assay known in the art or specifically described herein. Such functional derivatives can be made either by chemical peptide synthesis or chemical modification of protein known in the art of by recombinant means on the basis of the DNA sequences as disclosed herein by methods known in the state of the art and disclosed, e.g. by Sambrook et al (supra) ("Molecular Cloning" second edition, Cold Spring Harbour Laboratory Press 1989, New York). Amino acid exchanges in proteins and peptides which do not generally alter the activity of such molecules are known in the state of the art and are described, for example, by H. Neurath and R.L. Hill in "The Proteins" (Academic Press, New York, 1979, see especially Figure 6, page 14). The most commonly occurring exchanges are: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly as well as these in reverse.

The present invention is directed to recombinant DNA fragments which encode the said recombinant polypeptides involved in the said cytochrome c oxidase complex that is one of the essential components mediating electron transfer in the respiratory chain.

The recombinant DNA fragments which are useful for preparing the respective core subunits, i.e. COI, COII and COIII by genetic engineering are provided. Such recombinant polypeptides are useful for the components to be involved in the novel cytochrome c oxidase complex of the present invention.

A recombinant DNA fragment for COI may be a DNA fragment which encodes a polypeptide involved in cytochrome c oxidase complex and comprises a DNA sequence selected from the group consisting of:
(a) the DNA sequence identified by SEQ ID NO: 1, and
(b) the DNA sequences which encode polypeptides having an amino acid sequence identified by SEQ ID NO: 2 or amino acid sequences having 85 % or higher identity with the said amino acid sequence.

A recombinant DNA fragment for COII may be a DNA fragment which encodes a polypeptide involved in cytochrome c oxidase complex and contains a DNA sequence selected from the group consisting of:
(a) the DNA sequence identified by SEQ ID NO: 3, and
(b) the DNA sequences which encode polypeptides having an amino acid sequence identified by SEQ ID NO: 4 or amino acid sequences having 85 % or higher identity with the said amino acid sequence.

Moreover, a recombinant DNA fragment for COIII may be a DNA fragment which encodes a polypeptide involved in cytochrome c oxidase complex and contains one or more DNA sequence(s) selected from the group consisting of:
(a) the DNA sequence identified by SEQ ID NO: 5,
(b) the DNA sequence identified by SEQ ID NO: 7,
(c) the DNA sequences which encode polypeptides having an amino acid sequence identified by SEQ ID NO: 6 or amino acid sequences having 85 % or higher identity with the said amino acid sequence, and
(d) the DNA sequences which encode polypeptides having an amino acid sequence identified by SEQ ID NO: 8 or amino acid sequences having 85 % or higher identity with the said amino acid sequence.

The recombinant DNA fragment of this invetion may also include a DNA sequence which is capable of hybridizing to SEQ ID NO: 1, 3, 5, or 7 under standard stringency conditions which are described in more detail below.

"Standard conditions" for hybridization mean the condition which are generally used by a person skilled in the art to detect specific hybridization signals and which are described, e. g. by Sambrook et al (supra), or preferably so called stringent hybridization and non-stringent washing conditions or more preferably so called moderately stringent conditions or even more preferably so called stringent hybridization and stringent washing conditions a person skilled in the art is familiar with and which are described, e.g. in Sambrook et al (supra).

The present invention also provides an expression vector comprising one or more of the above mentioned recombinant DNA fragments, which vector is suitable for the expression in an organism, pro- or eukaryotic host cell. Such an expression vector can be constructed by inserting one or more of the above mentioned recombinant DNA fragments into a suitable vector which may carry expression control elements as it is well known in the art.

Further, a recombinant organism of the present invention may be prepared by introducing an expression vector mentioned above to an appropriate host cell. Such a recombinant organism of the invention will be useful for the genetic preparation of a recombinant cytochrome c oxidase complex of the present invention and also applicable to a process for producing 2KGA from L-sorbose or D-sorbitol in an appropriate culture medium. Host cells for the recombinant organism of the present invention maybe of eukaryotic origin, preferably a mammalian or plant cell, or may be of prokaryotic origin. These host cells may in particular be obtained from bacteria, preferably G. oxydans DSM No. 4025 and biologically and/or taxonomically homogeneous cultures of a microorganism having the identifying characteristics of G. oxydans DSM 4025. As well, host cells may be selected from the group consisting of bacteria, such as Escherichia coli, Pseudomonas putida, Acetobacter xylinum, Acetobacter pasteurianus, Acetobacter aceti, Acetobacter hansenii, and G. oxydans.

In addition it is an object of the present invention to provide a process for producing cytochrome c oxidase, which comprises cultivating a recombinant host cell as defined above in an appropriate culture medium and recovering the cytochrome c oxidase from the culture.

Cytochrome c oxidase complex of the present invention is also applicable for improving 2KGA production from L-sorbose or D-sorbitol and furthermore the production of aldehydes, carboxylic acids, and ketones from corresponding substrates in the presence of alcohol and aldehyde dehydrogenase in vivo and in vitro.

The compound 2KGA is an important intermediate for the production of L-ascorbic acid into which it can be converted according to the well-known Reichstein method. The production of 2KGA from L-sorbose or from D-sorbitol by fermentation is known [T. Hoshino et al., EP 88116156 A]. Gluconobacter strains are known to produce 2KGA via the reaction catalyzed by sorbose and sorbosone dehydrogenases as disclosed in Agric. Biol. Chem., 54(5), 1211-1218, 1990 [T. Hoshino et al.] and in EP 606621 A [T. Hoshino et al.]. The genes of primary dehydrogenases responsible for 2KGA formation from L-sorbose or D-sorbitol have been obtained [T. Hoshino et al., EP 832974 A]. Furthermore, cytochrome c functioning as an eletrone acceptor from the primary dehydrogenases and its gene has also been isolated [T. Hoshino et al., EP 869175 A]. These dehydrogenases and cytochrome c have been used to produce 2KGA in vitro. The genes have been used to construct recombinant organisms producing 2KGA from L-sorbose and D-sorbitol; e.g. Pseudomonas putida carrying the genes of alcohol/aldehyde dehydrogenase (AADH) together with cytochrome c can produce 2KGA from L-sorbose.

Therefore the use of cytochrome c oxidase of the present invention for the production of 2KGA is also an object of the present invention.

The terminal oxidase activity of the present cytochrome c oxidase complex was spectrophotometorically measured using TMPD (N,N,N',N'-tetramethyl-p-phenylenediamine dihydrochloride) as an artificial substrate (electron donor). The reaction mixture e.g., consists of 2.5 mM TMPD, 0.05% Tween-20 and 0.1 M sodium 3[N-morpholino]propanesulfonic acid (Na-MOPS) (pH 6.5). The TMPD oxidase activity can be measured by increasing of absorption at 520 nm, and mole coefficient of TMPD is taken as 6.1 /mM/cm. One unit of enzyme activity is defined as 1 micromole oxidation of TMPD per one minute at room temperature.

Spectrophotometoric identification and quantification of a-type heme were carried out by detection of characteristic positive peak around 605 nm by reduced minus oxidized difference spectrum. Reduction of each sample was done by addition of tiny amount of sodium dithionite, oxidation by ammonium persulfate. Mole coefficient of a-type heme peak (605 nm - 630 nm) was taken as 11.7 /mM/cm.

Before describing the present invention in more detail the physico-chemical properties of purified cytochrome c oxidase consisting of subunits, COI and COII, as obtainable from G. oxydans DSM 4025 are given below.

### (1) Absorption spectrum

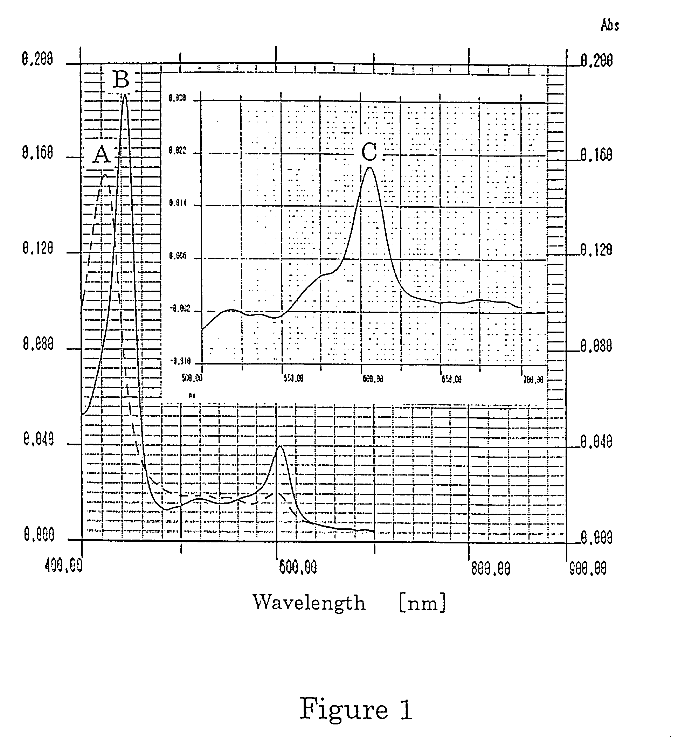

Absorption profile of the cytochrome c oxidase complex in reduced minus oxidized difference spectra is shown in Fig. 1.

### (2) Molecular weight

SDS-PAGE analysis showed apparent molecular masses of about 43 +/- 10 and 36 +/- 10 kDa for cytochrome c oxidase CO I and CO II subunits, respectively, as shown in Fig. 2.

### (3) Amino acid sequences of the CO I and CO II

The cytochrome c oxidase complex purified is dissociated into CO I and II subunits by a preparative-disc-SDS-PAGE (NA-1800, Nippon Eido Co,.). Both N-terminal alpha-amino residues were blocked by unidentified modification. Then, partially digested peptide fragments (15-45 kDa MW.) were derived by lysyl-endopeptidase treatment, isolated by band extraction from 15% SDS-PAGE sheet, washed in Centricon-10(Amicon) with 15% methanol and 0.1% SDS, and applied to the sequencer. "KDIGLLYLVAAGVVGF" (SEQ ID NO: 11) and "KASQFTHNTPLEIVWTIVPV" (SEQ ID NO: 14) sequences were obtained for CO I and COII, respectively.

A preferred strain used for isolating polypeptides and genes of cytochrome c oxidase of the present invention is G. oxydans strain that has been deposited at the Deutsche Sammlung von Mikroorganismen in Gottingen (Germany) under DSM 4025 on March 17, 1987 under the stipulations of the Budapest Treaty. Moreover, a subculture of the strain has also been deposited in the Agency of Industrial Science and Technology, Fermentation Research Institute, Japan, under the stipulations of the Budapest Treaty under the deposit No.: Gluconobacter oxydans FERM BP-3812 (date of deposit: March 30, 1992). Furthermore, EP 278 447 discloses the characteristics of this strain. Functional equivalents, subcultures, mutants and variants of said microorganism can be used in the present invention. Biologically or taxonomically homogeneous cultures of a microorganism having the identifying characteristics of the strain DSM 4025 can be also used as the source of the polypeptides and genes of the said cytochrome c oxidase.

The cytochrome c oxidase provided by the present invention can be prepared by cultivating an appropriate organism, disrupting the cells and isolating and purifying it from cell free extract of disrupted cells, preferably from the soluble fraction of the organism.

The organisms may be cultured in an aqueous medium supplemented with appropriate nutrients under aerobic conditions. The cultivation may be conducted at pH between about 4.0 and 9.0, preferably between about 6.0 and 8.0. While the cultivation period varies depending upon pH, temperature and nutrient medium used, usually 2 to 6 days will bring about favorable results. A preferred temperature range for carrying out the cultivation is from about 13° to 36°C, preferably from about 18° to 33°C.

It is usually required that the culture medium contains such nutrients as assimilable carbon sources, digestible nitrogen sources and inorganic substances, vitamins, trace elements and the other growth promoting factors. As assimilable carbon sources, glycerol, D-glucose, D-mannitol, D-fructose, D-arabitol, L-sorbose, D-sorbitol and the like can be used.

Various organic or inorganic substances may also be used as nitrogen sources, such as yeast extract, meat extract, peptone, casein, corn steep liquor, urea, amino acids, nitrates, ammonium salts and the like. As inorganic substances, magnesium sulfate, potassium phosphate, ferrous and ferric chlorides, calcium carbonate and the like may be used.

In the following, embodiments for the isolation and purification of cytochrome c oxidase from the organisms after the cultivation and for its cloning of the gene/DNA sequence are described.
(1) Cells are harvested from the fermentation broth by centrifugation or filtration.
(2) The cells are suspended in the buffer solution and disrupted by means of a homogenizer, sonicator or treatment with lysozyme and the like to give a disrupted solution of cells.
(3) Cytochrome c oxidase is isolated and purified from a cell free extract of disrupted cells, preferably from the soluble fraction of the organisms by usual protein purification methods such as ammonium sulfate precipitation, dialysis, ion exchange chromatographies, gel filtration chromatographies, and affinity chromatographies.

The cytochrome c oxidase provided by the present invention is useful as a terminal oxidase oxidizing cytochrome c, an electron acceptor from an enzyme belonging to dehydrogenase for the production of aldehydes, carboxylic acids and ketones from alcohols and aldehydes, especially for the production of 2KGA from L-sorbose or D-sorbitol via L-sorbosone.

Briefly, the cytochrome c oxidase genes, the DNA sequences, the recombinant expression vector and the recombinant organism, also called transformed host cell, utilized in the present invention can be obtained by the following steps:
(1) Isolating chromosomal DNA from the organisms which can provide cytochrome c oxidase of the present invention and constructing the gene library with the chromosomal DNA in Escherichia coli.
(2) Cloning cytochrome c oxidase genes from a chromosomal DNA by colony-, plaque-, or Southern-hybridization, PCR (polymerase chain reaction) cloning, Western-blot analysis and the like.
(3) Determining the nucleotide sequences of the cytochrome c oxidase genes obtained as above by usual methods to select recombinant DNA fragments containing said cytochrome c oxidase genes and constructing the expression vector on which cytochrome c oxidase genes can express efficiently.
(4) Constructing recombinant organisms carrying cytochrome c oxidase gene by transformation, transduction, transconjugation and electroporation.

The materials and the techniques used in the above aspect of the present invention are exemplified in details as follows:

A total chromosomal DNA can be purified by a procedure well known in the art. The genes encoding cytochrome c oxidase are cloned in either plasmid or phage vectors from a total chromosomal DNA by the following methods:
(i) by determining the partial amino acid sequences from the purified cytochrome c oxidase subunits by isolating the whole protein or peptide fragments obtained by peptidase-treatment from the gel after SDS-polyacrylamide gel electrophoresis and applying them to protein sequencer such as Applied Biosystems automatic gas-phase sequencer 470A (Perkin Elmer Corp., Norwalk, Conn., USA), synthesizing oligonucleotide probes with DNA synthesizer such as Applied Biosystems automatic DNA sequencer 381A (Perkin Elmer), according to the amino acid sequences obtained as above, isolating clones carrying the objective genes from a gene library of the strain carrying the objective genes with the oligonucleotide probes through Southern-, colony- or plaque-hybridization; (ii) by selecting clones expressing cytochrome c oxidase subunits from the gene library by immunological methods with antibodies against the subunits of cytochrome c oxidase; or (iii) by amplifying the DNAs from the total chromosomal DNA by PCR method with sets of two oligonucleotides synthesized according to the amino acid sequences determined as above and isolating clones carrying the whole genes of cytochrome c oxidase subunits from the gene library constructed in E. coli by Southern-, colony-, or plaque-hybridization with the PCR product obtained above as the probe. Above mentioned antibodies reacting against the subunits of cytochrome c oxidase can be prepared with the purified proteins of cytochrome c oxidase subunits or their peptide fragments by such method described in Methods in Enzymology, vol. 73, p 46, 1981.

The nucleotide sequences of the cytochrome c oxidase genes can be determined by a well-known method such as dideoxy chain termination method with M13 phage (Sanger F. et al., Proc. Natl. Acad. Sci. USA, 74:5463-5467, 1977).

To express the genes of cytochrome c oxidase complex subunits, various promoters can be used; for example, the original promoter existing upstream of the said genes for cytochrome c oxidase subunits, promoters of antibiotic resistance genes such as kanamycin resistant gene of Tn5 (Berg, D. E., and C. M. Berg. 1983. Bio/Technology 1: 417-435), ampicillin resistant gene of pBR322, and beta-galactosidase gene of E. coli (lac), trp-, tac-, trc-promoter, promoters of lambda phage and any promoters which can be functional in the hosts consisting of organism including bacteria such as E. coli, P. putida, A. xylinum, A. pasteurianus, A. aceti, A. hansenii, and G. oxydans, especially G. oxydans DSM 4025, mammalian cells and plant cells.

For the object above other regulatory elements such as a Shine-Dalgarno (SD) sequence (for example, AGGAGG etc. including natural and synthetic sequences operable in the host cell) and a transcriptional terminator (inverted repeat structure including any natural and synthetic sequence operable in the host cell) which are operable in the host cell into which the coding sequence will be introduced and used with the above described promoter.

A wide variety of host/cloning vector combinations may be employed in cloning the double-stranded DNA. Cloning vector is generally a plasmid or phage which contains an replication origin, regulatory elements, a cloning site including a multi-cloning site and selection markers such as antibiotic resistance genes including resistance genes for ampicillin, tetracycline, kanamycin, streptomycin, gentamicin, spectinomycin etc.

Preferred vectors for the expression of the object gene in E. coli are selected from any vectors usually used in E. coli, such as pBR322 or its derivatives including pUC18 and pBluescript II, pACYC177 and pACYC184 [J.Bacteriol., 134:1141-1156, 1978] and their derivatives, and a vector derived from a broad host range plasmid such as RK2 and RSF1010. A preferred vector for the expression of the object gene in Gluconobacter including G. oxydans DSM 4025 and P. putida is selected from any vectors which can replicate in Gluconobacter and/or P. putida, as well as a in preferred cloning organism such as E. coli. The preferred vector is a broad-host-range vector such as a cosmid vector like pVK102 and its derivatives and RSF1010 and its derivatives, and a vector containing a replication origin functional in Gluconobacter and another origin functional in E. coli. Copy number and stability of the vector should be carefully considered for stable and efficient expression of the cloned gene and also for efficient cultivation of the host cell carrying the cloned gene. DNA sequences containing transposable elements such as Tn5 can be also used as a vector to introduce the object gene into the preferred host, especially on a chromosome. DNA sequences containing any DNAs isolated from the preferred host together with the object gene are also useful to introduce the desired DNA sequence into the preferred host, especially on a chromosome. Such DNA sequences can be transferred to the preferred host by transformation, transduction, transconjugation or electroporation.

Useful hosts are of pro- or eukaryotic origin and may include organisms, mammalian cells, and plant cells. As a preferable organism, there may be mentioned bacteria such as E. coli, P. putida, A. xylinum, A. pasteurianus, A. aceti, A. hansenii, G. oxydans, and any Gram-negative bacteria which are capable of producing recombinant cytochrome c oxidase. Functional equivalents, subcultures, mutants and variants of said organism can be also used in the present invention. A preferred strain is E. coli K12 and its derivatives, P. putida or G. oxydans DSM 4025 and biologically or taxonomically homogeneous cultures of a microorganism having the identifying characteristics of strain DSM 4025.

The DNA sequence encoding cytochrome c oxidase of the present invention is ligated into a suitable vector containing a regulatory region such as a promoter and a ribosomal binding site and transcriptional terminator operable in the host cell described above by well-known methods in the art to produce an expression vector.

To construct a host cell carrying an expression vector, various DNA transfer methods including transformation, transduction, conjugal mating (Chapters 14 and 15, Methods for general and molecular bacteriology, Philipp Gerhardt et al. ed., American Society for Microbiology, 1994), and electroporation can be used. The method for constructing a transformed host cell may be selected from the methods well-known in the field of molecular biology. Usual transformation system can be used for E. coli, Pseudomonas and Acetobacter. Transduction system can also be used for E. coli. Conjugal mating system can be widely used in Gram-positive and Gram-negative bacteria including E. coli, P. putida and G. oxydans. A preferred conjugal mating method was basically disclosed in WO 89/06688. The conjugation can occur in liquid medium or on a solid surface. The preferred recipient for cytochrome c oxidase production is selected from E. coli, P. putida and G. oxydans. The preferred recipient for 2KGA production is selected from E. coli, P. putida and G. oxydans which can produce active AADHs and cytochrome c with a suitable recombinant expression vector. The preferred recipient for 2KGA production is G. oxydans DSM 4025. To the recipient for conjugal mating, a selective marker is usually added; for example, resistance against nalidixic acid or rifampicin is usually selected.

The Examples which follow are provided to further illustrate the invention and are not intended to limit the invention in any way.

### Example 1:

### Identification and purification of cytochrome c oxidase of G. oxydans DSM 4025

Terminal oxidase activity was spectrophotometorically measured using TMPD (N,N,N',N'-tetramethyl-p-phenylenediamine dihydrochloride) as an artificial substrate (electron donor). The reaction mixture consisted of 2.5 mM TMPD, 0.05% Tween-20 and 0.1 M sodium 3[N-morpholino]propanesulfonic acid (Na-MOPS) (pH 6.5). TMPD oxidase activity was measured by increasing of absorption at 520 nm, and mole coefficient of TMPD was taken as 6.1 /mM/cm. One unit of enzyme activity was defined as 1 micromole oxidation of TMPD per one minute at room temperature. Spectrophotometoric identification and quantification of a-type heme were carried out by analysing a reduced minus oxidized difference spectrum to detect the characteristic positive peak around 605 nm. Each sample was reduced with sodium dithionite and oxidized with ammonium persulfate. Mole coefficient of a-type heme peak (605 nm - 630 nm) was taken as 11.7 /mM/cm.

G. oxydans DSM 4025 was aerobically cultivated with 5 liter of FYC medium, which consisted of 10% L-sorbose (separately sterilized), 0.05% glycerol, 1.6% urea (separately sterilized), 0.25% MgSO₄x7H₂O, 6.25% baker's yeast cells, 1.5% CaCO₃ (production grade, nacalai tesque, Kyoto, Japan), and 3.0% corn steep liquor, pH 7.5 (before sterilization) at 30°C for 27 hours. After the cultivation, solid materials such as CaCO₃ and yeast cells were precipitated by low speed centrifugation (1,000 rpm for 5 minutes) and removed. G. oxydans DSM 4025 cells remained in the culture supernatant were collected by centrifugation at 8,000 rpm for 20 minutes and once washed with 25 mM sodium N-[2-hydroxyethyl] piperazine-N'-[4-butanesulfonic acid] (Na-HEPES) (pH 7.5) containing 0.25 M NaCl, and 2 mM MgCl₂.

The resulting cells (about 35 g wet weight) were suspended in about 200 ml of 25 mM Na-HEPES (pH 7.5) containing 0.5 mM ethylenediamine tetraacetic acid (EDTA), 0.5 mM ethylene glycol-bis-beta-aminoethyl ether (EGTA) 0.5 mM phenylmethylsulfonyl fluoride (PMSF), 1 microgram/ml pepstatin A, 1 microgram/ml leupeptin, 10 microgram/ml DNase I and 10 microgram/ml RNase A. The cell suspension was treated with French press homogenizer at 1500 kg/cm2 twice. The resulting suspension was centrifuged at 10,000 rpm for 10 minutes to remove cell debris, and the supernatant was collected as cell-free extract (424.0 mg proteins). The cell-free extract was subjected to ultra-centrifugation operated at 55000 rpm for 1 hour to recover the precipitate as a crude membrane fraction. The crude membrane fraction was resuspended in 50 ml of 25 mM Na-HEPES (pH 7.5) containing 1.2% Tween 20, 0.25 M NaCl, 2 mM MgCl₂, 0.5 mM PMSF, 1 microgram/ml pepstatin A and 1 microgram/ml leupeptin and incubated for 1 hour for washing the membrane. The fraction was again subjected to ultra-centrifugation operated at 55,000 rpm for 1 hour to recover the precipitate as a washed membrane fraction. The washed membrane fraction was incubated with 50 ml of 25 mM Na-HEPES (pH 7.5) containing 1.5% sucrose monolaulate (DOJIN Laboratories, Kumamoto, Japan), 2 mM EDTA and 5% glycerol for 1 hour to solubilize the membrane-bound proteins and the resulting suspension was subjected to ultra-centrifugation operated at 55,000 rpm for 1 hour to obtain a supernatant (50 ml) as solubilized membrane fraction. Reduced minus oxidized difference spectrum of the solubilized membrane fraction showed characteristic positive peak around 605 nm; the peak corresponds to 0.41 nmoles of a-type heme/mg of crude proteins as content. Then, membrane-bound proteins in the solubilized membrane fraction were load on a DEAE-Toyopearl 650M (TOSOH, Tokyo, Japan) column (ID 2.2 x 5 cm) which had been equilibrated with 25 mM Na-HEPES containing 0.5% sucrose monolaurate and 5% glycerol. Fractionation was carried out by a liner gradient of 0 - 0.35 M NaCl in the same buffer. Fractions showing a-type heme spectra (positive peak around 605 nm on reduced minus oxidized difference spectrum) and TMPD oxidase activity were eluted around 0.28 M concentration of NaCl. These fractions were collected (64 ml), dialyzed against 45 mM potassium phosphate buffer [KPB] (pH 7.6) containing 45 mM NaCl, 5% glycerol and 0.5% sucrose monolaurate, and the enzyme solution was loaded on hydroxylapatite (TONEN Co., Tokyo, Japan) column (ID 1.5 x 6 cm) which had been equilibrated with the same buffer. The column was washed with the same buffer and then with 500 mM KPB (pH 7.6) containing 500 mM NaCl, 5% glycerol and 0.5% sucrose monolaurate, and the active fractions were eluted with 900 mM KPB (pH 7.6) containing 900 mM NaCl, 5% glycerol and 0.5% sucrose monolaurate and collected. The fraction (8.6 mg protein) from the hydroxylapatite column was dialyzed against 25 mM Na-HEPES (pH 7.5) containing 0.5% sucrose monolaurate and 5% glycerol, concentrated by ultrafiltration using YM-30 membrane (Amicon Inc., MA, USA) and stored at -30°C as a purified protein.

The purified protein was subjected to native-polyacrylamide gel electrophoresis (Native-PAGE) analysis in the presence of 0.5% sucrose monolaurate; the protein showed a visible band with greenish color (without protein staining) which corresponded to a single protein band (with protein staining). The purified protein had 2.6 units/mg of TMPD oxidase activity and showed typical absorption spectra pattern of aa3-type cytochrome c oxidase (Fig. 1). Content of a-type heme was estimated to be 19.2 nmoles/mg of the purified protein. Purification fold on the content of a-type heme from the washed membrane was about 100-folds with nearly 90% recovery. These results indicated that the purified protein was a major component with TMPD-oxidase activity in G. oxydans DSM 4025, which can function as a terminal oxidase on respiratory system. In SDS-PAGE analysis, the purified protein was disassociated into two protein components: one showed a broad band with an apparent molecular weight of about 43,000 (named as CO I) and the other showed a sharp band with apparent molecular weight of about 36,000 (named as CO II). (Fig. 2)

### Example 2:

### Amino acid sequences of cytochrome c oxidase of G. oxydans DSM 4025 and the homologies with the other cytochrome c oxidase complexes.

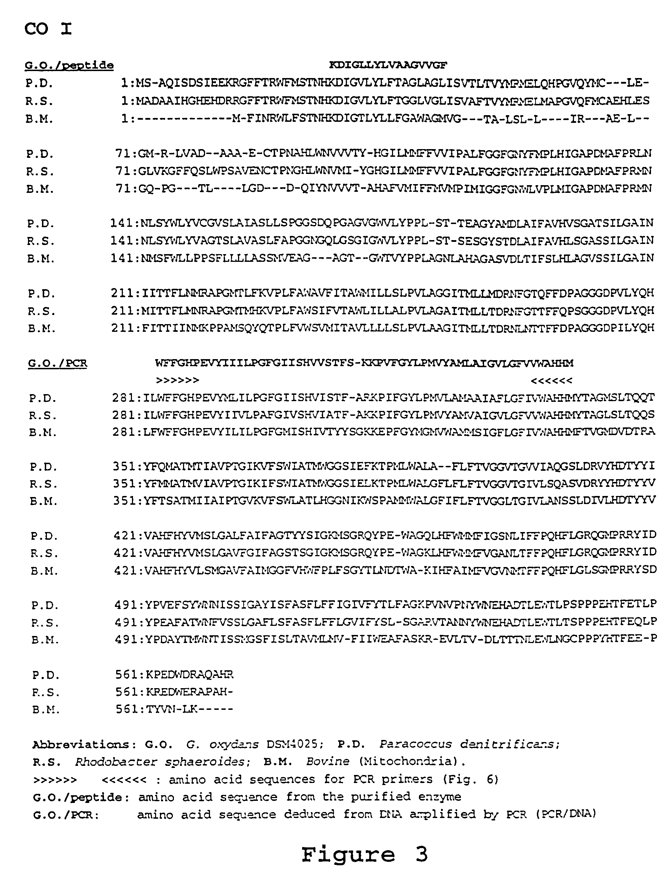

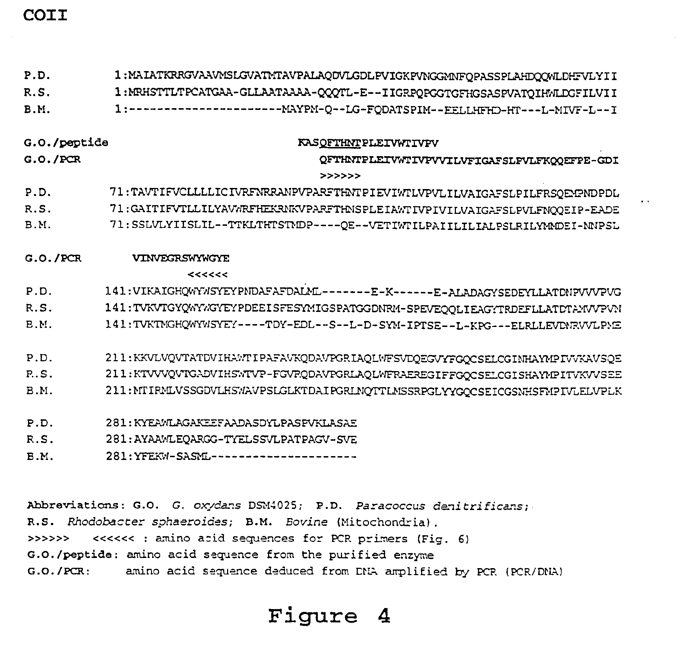

Two components (CO I and CO II) of the purified cytochrome c oxidase of G. oxydans DSM 4025 were disassociated by a preparative SDS-PAGE. From both components, native N-terminal amino acid sequences were not obtained. To obtain the internal amino acid sequence, each component was digested by lysyl-endopeptidase and the resulting fragments were isolated by preparative SDS-PAGE and subjected to amino acid sequencing with an amino acid sequencer (Applied Biosystems model 470A, The Perkin Elmer Corp., Conn., USA). As the results, partial amino acid sequences were obtained; KDIGLLYLVAAGVVGF [SEQ ID NO: 11], was obtained from the CO I fragment (slightly lower molecular weight from the original) and KASQFTHNTPLEIVWTIVPV [SEQ ID NO:14], was from the CO II fragment (about 10000 lower molecular weight from the original). The partial amino acid sequences of the CO I and CO II subunits were compared with the total amino acid sequence of cytochrome c oxidase complexes of P. denitrificans and R. sphaeroides and bovine mitochondria by sequence-alignments (Figs. 3 to 4) because the CO I and CO II were similar to those of cytochrome c oxidase of P. deni trificans (B. Ludwig and G. Schatz, Proc. Natl. Acad. Sci. USA, 77, 196-200, 1980) in the SDS-PAGE and spectrophotometric characters. Total homology in the amino acid sequences among three cytochrome c oxidase complexes had been previously reported (C. Jianli et al., J. Biol. Chem., 267, 24273-24278, 1992). As shown in Figs. 3 and 4, the amino acid sequences of G. oxydans DSM 4025 cytochrome c oxidase CO I and CO II were partially assigned to the homology alignment of the others. Especially, significant homology was observed with two bacterial sequences (P. denitrificans and R. sphaeroides).

### Example 3:

### Cloning of cytochrome c oxidase genes of G. oxydans DSM 4025

### (1) Amplification of partial cytochrome c oxidase gene(s) by the PCR method.

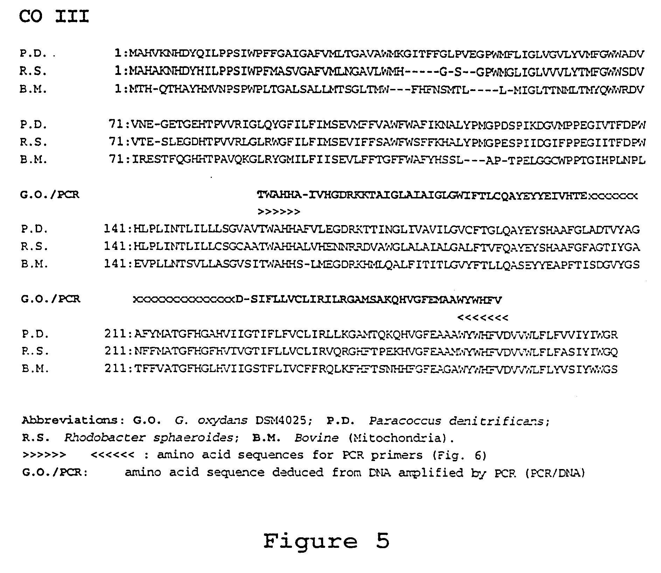

According to the total amino acid sequence alignments of P. denitrificans, R. sphaeroides and bovine mitochondria together with the amino acid sequences of the purified CO I and CO II polypeptides (SEQ ID NOs: 11 and 14), the following amino acid sequences were selected for PCR primers to amplify partial DNA sequences of CO I and CO II genes: SEQ ID NO: 9 and SEQ ID NO:10 for the CO I gene; and SEQ ID NO: 15 and SEQ ID NO:16 for the CO II gene. The third component (CO III), which was reported to be included in cytochrome c oxidase complex, did not exist in the preparation purified from G. oxydans DSM 4025; the absence seemed to be due to disassociation during the purification. To confirm and amplify a partial DNA sequence encoding the assumed CO III gene of G. oxydans DSM 4025, if it exists, two amino acid sequences, which were conserved in the polypeptides encoded by three CO III genes of P. denitrificans, R. sphaeroides and bovine mitochondria, were selected (Fig. 5.) : SEQ ID NO: 17 and SEQ ID NO:18 for CO III gene. Each pair of primers was designed for CO I, CO II or CO III (Fig. 6). The PCR reaction was carried out by using the GeneAmp^{TM} DNA Amplification Reagent Kit (Takara Shuzo, Kyoto, Japan) with the Parkin-Elmer Cetus Instruments Thermal Cycler according to the recommendations of the supplier. The reaction consisted of 30 cycles of 1) denaturation step at 94°C for 1 minute; 2) annealing step at 42 or 50°C for 2 minutes; and 3) synthesis step at 72°C for 3 minutes. The reaction mixture (100 microliter) contained 200 micromole of dNTPs, 2.9 micromole (for 32 degeneracy) or 5.8 micromole (for 64 degeneracy) of each primer, 2.2 ng of chromosomal DNA of G. oxydans DSM 4025, and 2.5 units of Taq polymerase in the buffer supplied. PCR product was detected by agarose gel electrophoresis [AGE] with ethidium bromide staining. As a result, DNA fragments with expected length (about 180 bp for CO I, about 180 bp for CO II, about 300 bp for CO III) were amplified.

### (2) Cloning and nucleotide sequencing of the DNA fragments amplified by PCR

The PCR-amplified DNA fragments were purified from an agarose gel and directly cloned into pCRTMII vector (Invitrogen Corporation, USA), and the DNA sequences were determined according to the supplier's instruction. Amino acid sequences deduced from the nucleotide sequences of the PCR products showed considerable homologies with the sequences of target positions in the alignments (Figs. 3 to 5). The PCR products encoding the partial amino acid sequences of CO I, CO II and CO III were labeled with ³²P to obtain probes Pcol, Pco2, and Pco3, respectively. The probes were used for Southern- or colony-hybridization to detect the complete CO I, CO II, and CO III genes.

### (3) Southern-blot analysis of the G. oxydans DSM 4025 chromosomal DNA using the PCR products as probes

The chromosomal DNA of G. oxydans DSM 4025 digested with various restriction endonucleases was subjected to Southern hybridization using the probes. The probe Pcol hybridized to a Pst I fragment (8.0 kb), and the probes Pco2 and Pco3 hybridized to an EcoRI fragment (9.3 kb).

### (4) Cloning of complete cytochrome c oxidase genes in the 8.0 kb PstI fragment (CO I) and the 9.3 kb EcoRI fragment (CO II and CO III)

The chromosomal DNA of G. oxydans DSM 4025 was completely digested with PstI or EcoRI and the resulting fragments were subjected to agarose gel electrophoresis. EcoRI-digests around 9.3 kb (7-12 kb) in size and PstI-digests around 8 kb (6-10 kb) were cut out and eluted from the gel. The recovered DNA fragments were ligated with PstI- or EcoRI-digested pUC19 vector to transform E. coli JM109. About 1,000 transformants were obtained as PstI- or EcoRI-library. Colony hybridization was performed with the probe Pcol for the PstI library and with the primers Pco2 and Pco3 for the EcoRI library. From each library, several positive colonies were obtained. Plasmid DNAs were extracted from the colonies and digested with PstI or EcoRI; 8.0 kb PstI fragment showed a strong signal with the probe Pcol, and 9.3 kb EcoRI fragment showed a strong signal with both of the probes Pco2 and Pco3. The plasmid containing 8.0 kb PstI fragment were designated as pUCO01 and the plasmid containing 9.3 kb EcoRI fragment as pUC023.

### (5) Physical map of the 8.0 kb PstI and 9.3 kb EcoRI fragments

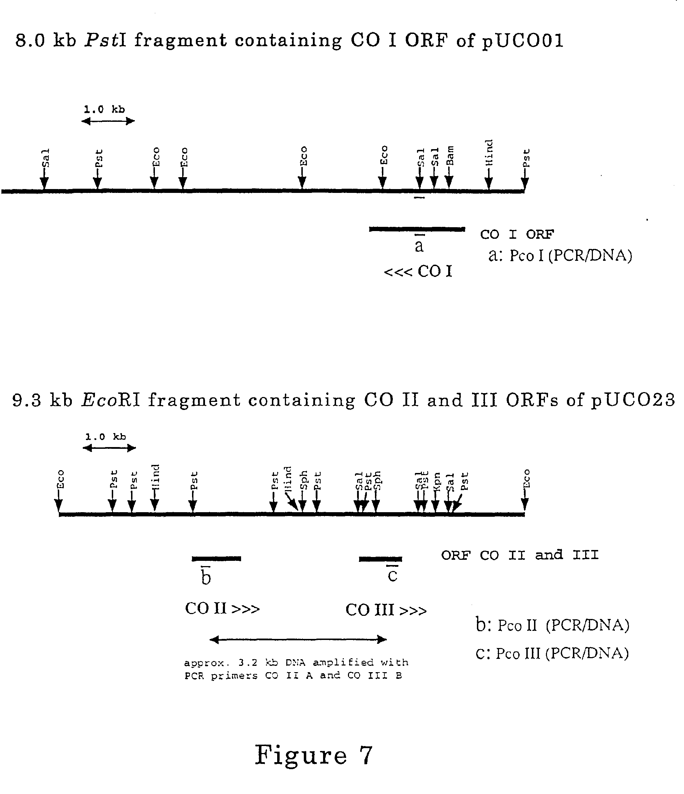

Physical maps of the 8.0 kb PstI and 9.3 kb EcoRI fragments were constructed by the Southern hybridization analysis of the fragments digested with various restriction endonucleases with the probes Pcol, Pco2 adn Pco3. Direction and distance of CO II and CO III genes encoded on the 9.3 kb EcoRI fragment was determined by the PCR method with primers derived from the partial nucleotide sequences (Fig. 7).

### (6) Nucleotide sequencing of the complete CO I gene

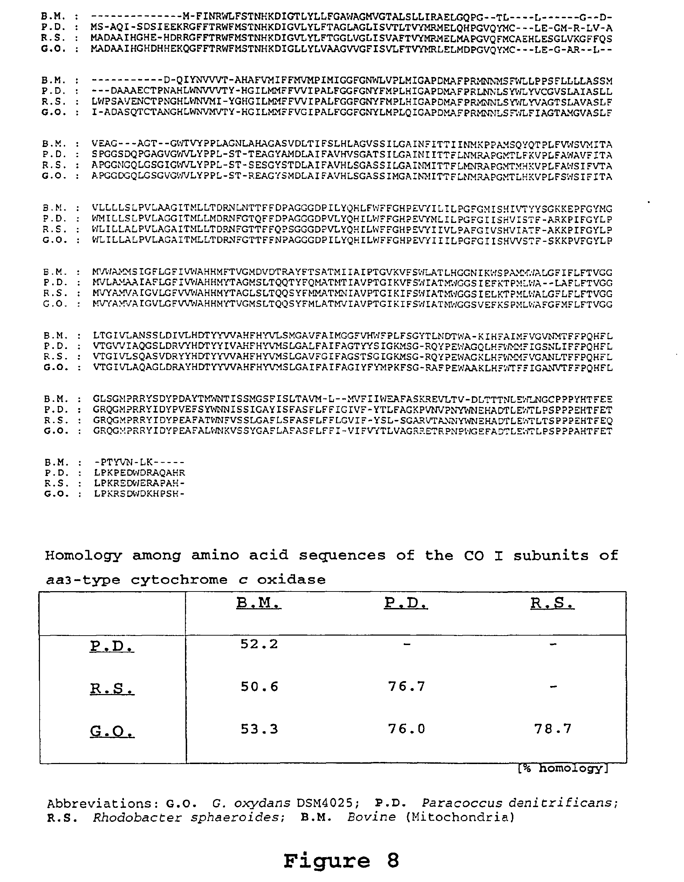

The nucleotide sequence of the COI genes on pUCO01 was determined by the dideoxy chain termination method. A 2.9 kb fragment upstream from HindIII site as shown in Fig. 7 was sequenced and one open reading frame (CDS of 1,674 bp existing in the sequence shown in SEQ ID NO: 1) was found in the fragment. This ORF encodes a protein of 558 amino acids (sequence list SEQ ID NO: 2), containing the stretch consistent with the amino acid sequence (SEQ ID NO: 11) of the peptide fragment derived from the purified CO I and the amino acid sequence (SEQ ID NO: 13) deduced from the DNA sequence of the about-180 bp PCR product (SEQ ID NO: 12) for CO I [see 3-(1)] The CO I amino acid sequence of G. oxydans DSM 4025 showed 78.7, 76.0 and 53.3% homologies with those of R. sphaeroides, P. denitrificans and bovine mitochondria, respectively. (Fig. 8)

### (7) Construction of an expression plasmid encoding all of CO I, CO II, and CO III genes

CO I gene was isolated from the 8.0 kb PstI fragment on pUCO01 by complete HindIII- and partail-EcoRI digestions as a 3.5 kb fragment (Fig. 7). According to the physical map of the 9.3 kb EcoRI fragment on pUC023, CO II and CO III genes were isolated by complete-KpnI and partial-PstI digestions to make a 6.0 kb fragment in tandem form (Fig. 7). Each fragment was once subcloned into a pBluescriptII SK+ vector to obtain plasmids pBCO01 with 3.5 kb fragment containing CO I gene and pBCO23 with 6.0 kb fragment containing CO II and CO III genes.

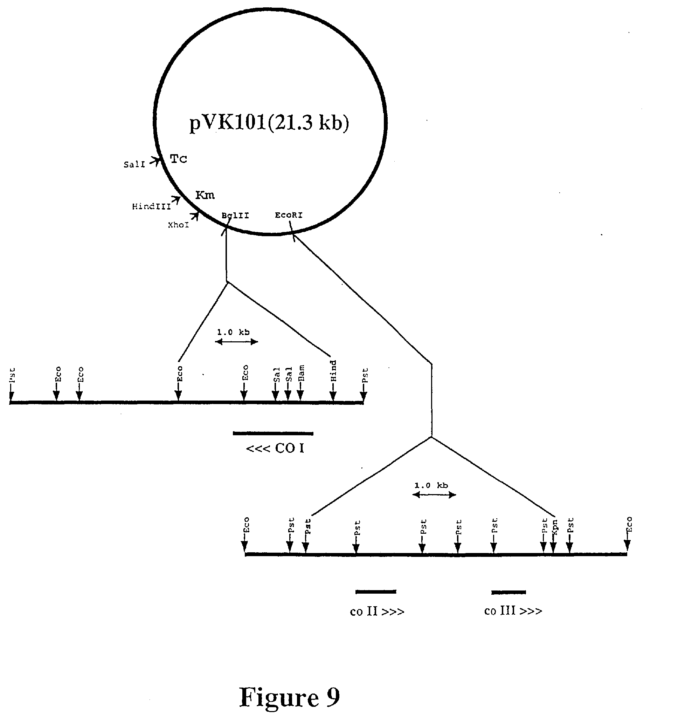

As shown in Fig. 9, the 3.5 kb fragment containing CO I gene and the 6.0 kb fragment containing CO II and CO III genes were co-integrated in one expression vector for functional expression of genes of the cytochrome c oxidase complex (CO I, CO II, and CO III). At first, the 6.0 kb Xbal - Kpnl fragment containing CO II and CO III genes from pBC023 was inserted in the EcoRI site of plasmid vector, pVK101 by the blunt end ligation. Then, the 3.5 kb Xbal - HindIII fragment containing CO I gene from pBCO01 was inserted in the BgIII site of pVK101 with the 6.0 kb Xbal - Kpnl fragment. The resulting plasmid vector was designated as pVKcoxes.

### Example 4:

### Overexpression of cytochrome c oxidase genes in G. oxydans DSM 4025 derivative

The plasmid carrying the cytochrome c oxidase genes in pVK101, pVKcoxes, was introduced into a rifampicin resistant derivative of G. oxydans DSM 4025, GOS2RPM [a single colony isolate from GOS2R; T. Hoshino et al., European Patent Publication 832974 A2] by the tri-parental conjugal mating method. Cells of GOS2RPM were cultivated at 30°C in 10 ml of T medium consisted of 3% Trypticase Soy Broth (Bacton Dickinson, Cockeysville, Md., USA) and 0.3% yeast extract (Difco Laboratories, Detroit, Mich.) with 100 microgram/ml of rifampicin (TR medium). A donor strain, E. coli HB carrying pVKcoxes (Tcr, Kmr) or pVK102 (Tcr, Kmr) and a helper strain, E. coli HB101 carrying pRK2013 (Kmr) were grown in Luria Bertani medium containing appropriate antibiotics overnight at 37°C. These overnight cultures (10 ml of GOS2RPM culture and 2 ml of E. coli culture) were independently centrifuged and cell pellets were independently suspended in 2 ml of T medium. One hundred microliter of the cell suspensions were mixed and 50 microliter of the mixed cell suspension were spotted onto a nitrocellulose filter placed on the surface of NS2 agar medium consisted of 5.0% D-mannitol, 0.25% MgSO4.7H2O, 1.75% corn steep liquor, 5% baker's yeast (Oriental Yeast Co., Tokyo, Japan), 0.5% CaC03, 0.5% urea (separately sterilized), and 2.0% agar, pH 7.0 (before sterilization). The plate was incubated at 27°C overnight. The resulting cells were spread onto T agar medium containing 100 microgram/ml rifampicin and 3 microgram/ml tetracycline (TRT agar plate). The transconjugants thus obtained were purified by streaking on TRT agar plate to remove cells of E. coli and plasmid-free GOS2RPM.

The resulting transconjugants, GOS2RPM (pVKcoxes) and GOS2R (pVK102) were cultivated, and cells of both transconjugants were prepared according to the method described in Example 1. Cytochrome c oxidase level in GOS2RPM (pVKcoxes) was determined by the following experiments in comparison with that of GOS2RPM (pVK102). From both strains, solubilized membrane fraction was prepared by the method described in Example 1. First, a-type heme contents were determined to be 0.031 and 0.022 nmoles/mg of cell proteins for GOS2RPM (pVKcoxes) and GOS2R (pVK102), respectively, by the reduced minus oxidized difference spectrum method (Example 1). Second, specific oxidation rate of cytochrome c (purchased from Sigma, horse heart type VI) was measured. Reduced cytochrome c was prepared by sodium dithinite as a reducer, and excess reducer was removed by twice treatment of PD-10 column (Pharmacia). Reaction mixture consisted of 33 mM reduced cytochrome c, 25 mM Na-HEPES (pH 7.2), 2% sucrose monolaurate and 0.5 mM EDTA. Oxidation rate of the reduced cytochrome c was measured by decrease of absorbance at 550 nm, and mole coefficient was taken as 21.1 /mM/cm. Specific oxidation rates on the reduced cytochrome c were determined to be 1.58 and 2.00 nmoles/mg cell proteins/min for GOS2RPM (pVK102) and GOS2R (pVKcoxes), respectively. Third, contents of CO I and CO II components were compared by the Western-blot analysis with the antibody against the component CO I or CO II. Stronger band intenses (60% increase for CO I and 41% increase for CO II by using CCD camera) were observed on GOS2RPM (pVKcoxes). These results suggested that introduction of pVKcoxes resulted in functional amplification of the cytochrome c oxidase complex level in the 2KGA producing G. oxydans DSM 4025 derivative.

## Claims

1. Cytochrome c oxidase complex having cytochrome c oxidase activity obtainable or obtained from biological or genetic material originated from a microorganism identified as Gluconobacter oxydans DSM 4025 or biologically and/or taxonomically homogeneous cultures of a microorganism having the identifying characteristics of Gluconobacter oxydans DSM 4025.

2. Cytochrome c oxidase complex as set forth in claim 1, which shows the following physicochemical properties of:
(i) showing the presence of at least two core subunits of I (COI) and II (COII) wherein; apparent molecular mass of COI is about 43 +/- 10 kDa by SDS-PAGE analysis; and apparent molecular mass of COII is about 36 +/- 10 kDa by SDS-PAGE analysis and
(ii) absorption spectrum showing aa3-type cytochrome c oxidase being; 605 +/- nm peak in reduced minus oxidized difference spectrum.

3. Cytochrome c oxidase complex as set forth in claim 1 or 2, which is substantially homogeneous isolate derived from a culture of a microorganism identified as Gluconobacter oxydans DSM 4025 or biologically and/or taxonomically homogeneous cultures of a microorganism having the identifying characteristics of Gluconobacter oxydans DSM 4025.

4. Cytochrome c oxidase complex as set forth in any one of claims 1 - 3, which is a recombinant enzyme.

5. Cytochrome c oxidase complex as set forth in claim 4, which comprises a recombinant polypeptide selected from the group consisting of polypeptides having an amino acid sequence identified by SEQ ID NO: 2 and those having amino acid sequences having 85% or higher identity with the said sequence and being capable of providing the complex with cytochrome c oxidase activity.

6. Cytochrome c oxidase complex as set forth in claim 4 or 5, which contains at least one recombinant polypeptide(s) selected from the group consisting of those containing amino acid sequences identified by SEQ ID NOs: 4, 6 and/or 8 and those containing amino acid sequences having 85% or higher identity with any one of the said amino acid sequences, and are capable of providing the complex with cytochrome c oxidase activity.

7. A recombinant polypeptide which is involved in cytochrome c oxidase complex described in any one of claims 1 - 6, which polypeptide comprises an amino acid sequence identified by SEQ ID NO: 2 or an amino acid sequence having 85 % or higher identity with the said amino acid sequence and is capable of providing the complex with cytochrome c oxidase activity.

8. A recombinant polypeptide as set forth in claim 7 capable of providing the complex described in any one of claims 1 - 6 with cytochrome c oxidase activity, which is encoded by a recombinant DNA fragment comprising a DNA sequence selected from the group consisting of:
(a) the DNA sequence identified by SEQ ID NO: 1, and
(b) the DNA sequences which encode polypeptides having an amino acid sequence identified by SEQ ID NO: 2 or amino acid sequences having 85 % or higher identity with the said amino acid sequence.

9. A recombinant polypeptide which is involved in cytochrome c oxidase complex described in any one of claims 1 - 6, which polypeptide contains an amino acid sequence identified by SEQ ID NO: 4, or an amino acid sequence having 85 % or higher identity with the said amino acid sequence and is capable of providing the said complex with cytochrome c oxidase activity.

10. A recombinant polypeptide as set forth in claim 9 capable of providing the complex described in any one of claims 1 - 6 with cytochrome c oxidase activity, which is encoded by a recombinant DNA fragment containing a DNA sequence selected from the group consisting of:
(a) the DNA sequence identified by SEQ ID NO: 3, and
(b) the DNA sequences which encode polypeptides having an amino acid sequence identified by SEQ ID NO: 4 or amino acid sequences having 85 % or higher identity with the said amino acid sequence.

11. A recombinant polypeptide which is involved in cytochrome c oxidase complex described in any one of claims 1 - 6, which polypeptide contains an amino acid sequence identified by SEQ ID NO: 6 or 8, or an amino acid sequence having 85 % or higher identity with either of the said amino acid sequences of SEQ ID NOs: 6 and 8 and is capable of providing the said complex with cytochrome c oxidase activity.

12. A recombinant polypeptide as set forth in claim 11 capable of providing the complex described in any one of claims 1 - 6 with cytochrome c oxidase activity, which is encoded by a recombinant DNA fragment containing a DNA sequence selected from the group consisting of:
(a) the DNA sequence identified by SEQ ID NO: 5,
(b) the DNA sequence identified by SEQ ID NO: 7,
(c) the DNA sequences which encode polypeptides having an amino acid sequence identified by SEQ ID NO: 6 or amino acid sequences having 85 % or higher identity with the said amino acid sequence, and
(d) the DNA sequences which encode polypeptides having an amino acid sequence identified by SEQ ID NO: 8 or amino acid sequences having 85 % or higher identity with the said amino acid sequence.

13. A recombinant DNA fragment, which comprises a DNA sequence claimed in claim 8 and which encodes an amino acid sequence of a polypeptide capable of providing the complex described in any one of claims 1-6 with cytochrome c oxidase activity, wherein the said DNA sequence is selected from the group consisting of:
(a) the DNA sequence identified by SEQ ID NO: 1, and
(b) the DNA sequences which encode polypeptides having an amino acid sequence identified by SEQ ID NO: 2 or amino acid sequences having 85 % or higher identity with the said amino acid sequence.

14. A recombinant DNA fragment, which comprises a DNA sequence claimed in claim 10 and which encodes an amino acid sequence contained in a polypeptide capable of providing the complex described in any one of claims 1-6 with cytochrome c oxidase activity, wherein the said DNA sequence is selected from the group consisting of:
(a) the DNA sequence identified by SEQ ID NO: 3, and
(b) the DNA sequences which encode polypeptides having an amino acid sequence identified by SEQ ID NO: 4 or amino acid sequences having 85 % or higher identity with the said amino acid sequence.

15. A recombinant DNA fragment, which comprises a DNA sequence claimed in claim 12 and which encodes an amino acid sequence contained in a polypeptide capable of providing the complex described in any one of claims 1-6 with cytochrome c oxidase activity, wherein the said DNA sequence is selected from the group consisting of: (a) the DNA sequence identified by SEQ ID NO: 5,
(b) the DNA sequence identified by SEQ ID NO: 7,
(c) the DNA sequences which encode polypeptides having an amino acid sequence identified by SEQ ID NO: 6 or amino acid sequences having 85 % or higher identity with the said amino acid sequence, and
(d) the DNA sequences which encode polypeptides having an amino acid sequence identified by SEQ ID NO: 8 or amino acid sequences having 85 % or higher identity with the said amino acid sequence.

16. An expression vector comprising one or more recombinant DNA fragments selected from the fragments claimed in claims 13, 14, and 15 wherein the expression vector is suitable for expression in an organism.

17. An expression vector according to claim 16, wherein the organism is a microorganism selected from the group consisting of bacteria such as Escherichia coli, Pseudomonas putida, Acetobacter xylinum, Acetobacter pasteurianus, Acetobacter aceti, Acetobacter hansenii and Gluconobacter oxydans.

18. An expression vector according to claim 16, wherein the organism is a microorganism identified as Gluconobacter oxydans DSM 4025 or biologically and/or taxonomically homogeneous cultures of a microorganism having the identifying characteristics of Gluconobacter oxydans DSM 4025.

19. A recombinant organism which is introduced by an expression vector claimed in any one of claims 16 - 18.

20. A recombinant organism as claimed in claim 18, in which the recombinant organism carries one or more DNA fragments selected from those claimed in claim 13, 14 and 15 integrated into its genome.

21. A recombinant organism according to claim 19 or 20, wherein a host organism is a microorganism selected from the group consisting of bacteria such as Escherichia coli, Pseudomonas putida, Acetobacter xylinum, Acetobacter pasteurianus, Acetobacter aceti, Acetobacter hansenii and Gluconobacter oxydans.

22. A recombinant organism according to claim 19 or 20, wherein a host organism is a microorganism identified as Gluconobacter oxydans DSM 4025 or biologically and/or taxonomically homogeneous cultures of a microorganism having the identifying characteristics of Gluconobacter oxydans DSM 4025.

23. A process for producing cytochrome c oxidase complex, which comprises cultivating a recombinant organism defined in any one of claims 19 - 22 in an appropriate culture medium and recovering the cytochrome c oxidase from the culture.

24. A process according to claim 23, wherein a host organism is a microorganism selected from the group consisting of bacteria such as Escherichia coli, Pseudomonas putida, Acetobacter xylinum, Acetobacter pasteurianus, Acetobacter aceti, Acetobacter hansenii and Gluconobacter oxydans.

25. A process according to claim 23, wherein a host organism is a microorganism identified as Gluconobacter oxydans DSM 4025 or biologically and/or taxonomically homogeneous cultures of a microorganism having the identifying characteristics of Gluconobacter oxydans DSM 4025.

26. A process for producing 2-keto-L-gulonic acid from L-sorbose or D-sorbitol, which comprises cultivating a recombinant organism defined in any one of claims 19 - 22 in an appropriate culture medium and recovering 2-keto-L-gulonic acid from the culture.

27. A process according to claim 26, wherein a host organism is a microorganism selected from the group consisting of bacteria such as Escherichia coli, Pseudomonas putida, Acetobacter xylinum, Acetobacter pasteurianus, Acetobacter aceti, Acetobacter hansenii and Gluconobacter oxydans.

28. A process according to claim 26, wherein a host organism is a microorganism identified as Gluconobacter oxydans DSM 4025 or biologically and/or taxonomically homogeneous cultures of a microorganism having the identifying characteristics of Gluconobacter oxydans DSM 4025.


SEQUENCE LISTING

<110> F. HOFFMANN-LA ROCHE AG

<120> Cytochrome c oxidase and its genes

<130> 20511 EP

<140>
<141>

<150> 99.122842.0
<151> 1999-11-17

<160> 18

<170> PatentIn Ver. 2.0

<210> 1
<211> 1674
<212> DNA
<213> Gluconobacter oxydans

<220>
<221> CDS
<222> (1)..(1674)

<400> 1

atg gca gac gcc gcc att cac ggc cat gac cac cat gag aag caa ggc 48

Met Ala Asp Ala Ala Ile His Gly His Asp His His Glu Lys Gln Gly

1               5                   10                  15

ttc ttc acg cgc tgg ttc atg tcg acc aac cac aaa gac atc ggt ctg 96

Phe Phe Thr Arg Trp Phe Met Ser Thr Asn His Lys Asp Ile Gly Leu

            20                  25                  30

cta tac ctt gta gcg gct ggt gtt gtt ggt ttc att tcc gtc ctg ttc 144

Leu Tyr Leu Val Ala Ala Gly Val Val Gly Phe Ile Ser Val Leu Phe

        35                  40                  45

acc gtc tac atg cgc ctt gag ctg atg gat ccg ggt gtt cag tac atg 192

Thr Val Tyr Met Arg Leu Glu Leu Met Asp Pro Gly Val Gln Tyr Met

    50                  55                  60

tgc ctt gaa ggc gca cgt ctg atc gcg gat gcc tcg cag aca tgt acg 240
Cys Leu Glu Gly Ala Arg Leu Ile Ala Asp Ala Ser Gln Thr Cys Thr
65                  70                  75                  80

gcg aac gga cac ctg tgg aac gtc atg gtt acc tac cat ggt att ctg 288
Ala Asn Gly His Leu Trp Asn Val Met Val Thr Tyr His Gly Ile Leu
                85                  90                  95

atg atg ttc ttt gtg ggt atc ccc gca ttg ttc ggt ggt ttt ggt aac 336
Met Met Phe Phe Val Gly Ile Pro Ala Leu Phe Gly Gly Phe Gly Asn
            100                 105                 110

tat ctg atg ccg ctg caa atc ggc gct ccg gat atg gcc ttc ccg cgt 384
Tyr Leu Met Pro Leu Gln Ile Gly Ala Pro Asp Met Ala Phe Pro Arg
        115                 120                 125

atg aac aac ctg tcg ttc tgg ctg ttc att gcc ggt acc gcg atg ggc 432
Met Asn Asn Leu Ser Phe Trp Leu Phe Ile Ala Gly Thr Ala Met Gly
    130                 135                 140

gtg gct tcg ctg ttc gca ccg ggc ggt gac ggt cag ctg ggt tcg ggc 480
Val Ala Ser Leu Phe Ala Pro Gly Gly Asp Gly Gln Leu Gly Ser Gly
145                 150                 155                 160

gtt ggt tgg gtt ctg tac ccg ccg ctg tcg acc cgc gaa gct ggc tat 528
Val Gly Trp Val Leu Tyr Pro Pro Leu Ser Thr Arg Glu Ala Gly Tyr
                165                 170                 175

tcg atg gac ctc gcg att ttc gcg gtt cac ttg tcg ggt gcc tcc tcg 576
Ser Met Asp Leu Ala Ile Phe Ala Val His Leu Ser Gly Ala Ser Ser
            180                 185                 190

atc atg ggc gcg atc aac atg atc acg acc ttc ttg aac atg cgc gcc 624
Ile Met Gly Ala Ile Asn Met Ile Thr Thr Phe Leu Asn Met Arg Ala
        195                 200                 205

ccc ggc atg acg ctg cac aaa gtg ccg ttg ttc tcg tgg tcg atc ttt 672
Pro Gly Met Thr Leu His Lys Val Pro Leu Phe Ser Trp Ser Ile Phe
    210                 215                 220

atc acg gct tgg ctg atc ctg ctg gcg ctg ccg gtt ctg gct ggt gca 720
Ile Thr Ala Trp Leu Ile Leu Leu Ala Leu Pro Val Leu Ala Gly Ala
225                 230                 235                 240

atc acc atg ctg ctg acc gac cgt aac ttc ggc acg acc ttc ttc aat 768
Ile Thr Met Leu Leu Thr Asp Arg Asn Phe Gly Thr Thr Phe Phe Asn
                245                 250                 255

cct gct ggc ggc ggt gac ccg att ctg tac caa cac atc ctg tgg ttc 816
Pro Ala Gly Gly Gly Asp Pro Ile Leu Tyr Gln His Ile Leu Trp Phe
            260                 265                 270

ttt ggg cac ccg gaa gtg tac atc atc att ctg ccc ggc ttt ggc atc 864
Phe Gly His Pro Glu Val Tyr Ile Ile Ile Leu Pro Gly Phe Gly Ile
        275                 280                 285

atc agc cat gtc gtg tcg acc ttc tcg aaa aag ccg gtc ttc ggt tac 912
Ile Ser His Val Val Ser Thr Phe Ser Lys Lys Pro Val Phe Gly Tyr
    290                 295                 300

ctg ccg atg gtc tat gca atg gtg gca atc ggt gtt ctg ggc ttt gtc 960
Leu Pro Met Val Tyr Ala Met Val Ala Ile Gly Val Leu Gly Phe Val
305                 310                 315                 320

gtc tgg gcg cac cac atg tac acc gtt ggt atg tcg ctg acc cag caa 1008
Val Trp Ala His His Met Tyr Thr Val Gly Met Ser Leu Thr Gln Gln
                325                 330                 335

tcc tac ttc atg ctg gcc acc atg gtg atc gcg gtg ccg acc ggc att 1056
Ser Tyr Phe Met Leu Ala Thr Met Val Ile Ala Val Pro Thr Gly Ile
            340                 345                 350

aag atc ttc tcg tgg atc gcc acg atg tgg ggc ggc tcg gtt gag ttc 1104
Lys Ile Phe Ser Trp Ile Ala Thr Met Trp Gly Gly Ser Val Glu Phe
        355                 360                 365

aaa tcg ccg atg ctc tgg gcc ttt ggc ttt atg ttc ctg ttc acc gtg 1152
Lys Ser Pro Met Leu Trp Ala Phe Gly Phe Met Phe Leu Phe Thr Val
    370                 375                 380

ggt ggt gtg acc ggt atc gtg ctg gcc caa gcg ggt ctg gac cgt gca 1200
Gly Gly Val Thr Gly Ile Val Leu Ala Gln Ala Gly Leu Asp Arg Ala
385                 390                 395                 400

tat cac gac acc tat tac gtg gtg gcg cac ttc cat tat gtg atg tcg 1248
Tyr His Asp Thr Tyr Tyr Val Val Ala His Phe His Tyr Val Met Ser
                405                 410                 415

ctg ggt gcg atc ttt gcg atc ttc gcc ggt atc tac ttt tac atg ccg 1296
Leu Gly Ala Ile Phe Ala Ile Phe Ala Gly Ile Tyr Phe Tyr Met Pro
            420                 425                 430

aag ttc tcg ggc cgc gct ttc ccg gaa tgg gct gca aag ctg cac ttc 1344
Lys Phe Ser Gly Arg Ala Phe Pro Glu Trp Ala Ala Lys Leu His Phe
        435                 440                 445

tgg acc ttc ttc atc ggt gcg aac gtc acg ttc ttc ccg cag cac ttc   1392
Trp Thr Phe Phe Ile Gly Ala Asn Val Thr Phe Phe Pro Gln His Phe
    450                 455                 460

ctg gga cgt cag ggt atg ccg cgc cgt tac atc gac tat ccc gaa gcc   1440
Leu Gly Arg Gln Gly Met Pro Arg Arg Tyr Ile Asp Tyr Pro Glu Ala
465                 470                 475                 480

ttc gcg ctg tgg aac aaa gtc tcg tcc tat ggt gcg ttc ctg gcc ttc   1488
Phe Ala Leu Trp Asn Lys Val Ser Ser Tyr Gly Ala Phe Leu Ala Phe
                485                 490                 495

gcc tcg ttc ctg ttc ttc atc gtg atc ttt gtc tat acg ctg gtt gct   1536
Ala Ser Phe Leu Phe Phe Ile Val Ile Phe Val Tyr Thr Leu Val Ala
            500                 505                 510

ggc cgc cgc gag acc cgt ccg aac ccg tgg ggc gaa ttc gcc gat acg   1584
Gly Arg Arg Glu Thr Arg Pro Asn Pro Trp Gly Glu Phe Ala Asp Thr
        515                 520                 525

ctg gaa tgg acg ctg cca tca ccg cct ccg gcc cac acg ttc gaa acg   1632
Leu Glu Trp Thr Leu Pro Ser Pro Pro Pro Ala His Thr Phe Glu Thr
    530                 535                 540

ctg ccc aag cgc tcg gac tgg gac aag cat ccc tcg cac taa           1674
Leu Pro Lys Arg Ser Asp Trp Asp Lys His Pro Ser His
545                 550                 555

<210> 2
<211> 557
<212> PRT
<213> Gluconobacter oxydans

<400> 2

Met Ala Asp Ala Ala Ile His Gly His Asp His His Glu Lys Gln Gly

1               5                   10                  15

Phe Phe Thr Arg Trp Phe Met Ser Thr Asn His Lys Asp Ile Gly Leu

            20                  25                  30

Leu Tyr Leu Val Ala Ala Gly Val Val Gly Phe Ile Ser Val Leu Phe

        35                  40                  45

Thr Val Tyr Met Arg Leu Glu Leu Met Asp Pro Gly Val Gln Tyr Met

    50                  55                  60

Cys Leu Glu Gly Ala Arg Leu Ile Ala Asp Ala Ser Gln Thr Cys Thr

65                  70                  75                  80

Ala Asn Gly His Leu Trp Asn Val Met Val Thr Tyr His Gly Ile Leu

                85                  90                  95

Met Met Phe Phe Val Gly Ile Pro Ala Leu Phe Gly Gly Phe Gly Asn

            100                 105                 110

Tyr Leu Met Pro Leu Gln Ile Gly Ala Pro Asp Met Ala Phe Pro Arg

        115                 120                 125

Met Asn Asn Leu Ser Phe Trp Leu Phe Ile Ala Gly Thr Ala Met Gly

    130                 135                 140

Val Ala Ser Leu Phe Ala Pro Gly Gly Asp Gly Gln Leu Gly Ser Gly

145                 150                 155                 160

Val Gly Trp Val Leu Tyr Pro Pro Leu Ser Thr Arg Glu Ala Gly Tyr

                165                 170                 175

Ser Met Asp Leu Ala Ile Phe Ala Val His Leu Ser Gly Ala Ser Ser
            180                 185                 190
Ile Met Gly Ala Ile Asn Met Ile Thr Thr Phe Leu Asn Met Arg Ala
        195                 200                 205
Pro Gly Met Thr Leu His Lys Val Pro Leu Phe Ser Trp Ser Ile Phe
    210                 215                 220
Ile Thr Ala Trp Leu Ile Leu Leu Ala Leu Pro Val Leu Ala Gly Ala
225                 230                 235                 240
Ile Thr Met Leu Leu Thr Asp Arg Asn Phe Gly Thr Thr Phe Phe Asn
                245                 250                 255
Pro Ala Gly Gly Gly Asp Pro Ile Leu Tyr Gln His Ile Leu Trp Phe
            260                 265                 270
Phe Gly His Pro Glu Val Tyr Ile Ile Ile Leu Pro Gly Phe Gly Ile
        275                 280                 285
Ile Ser His Val Val Ser Thr Phe Ser Lys Lys Pro Val Phe Gly Tyr
    290                 295                 300
Leu Pro Met Val Tyr Ala Met Val Ala Ile Gly Val Leu Gly Phe Val
305                 310                 315                 320
Val Trp Ala His His Met Tyr Thr Val Gly Met Ser Leu Thr Gln Gln
                325                 330                 335

Ser Tyr Phe Met Leu Ala Thr Met Val Ile Ala Val Pro Thr Gly Ile
            340                 345                 350
Lys Ile Phe Ser Trp Ile Ala Thr Met Trp Gly Gly Ser Val Glu Phe
        355                 360                 365
Lys Ser Pro Met Leu Trp Ala Phe Gly Phe Met Phe Leu Phe Thr Val
    370                 375                 380
Gly Gly Val Thr Gly Ile Val Leu Ala Gln Ala Gly Leu Asp Arg Ala
385                 390                 395                 400
Tyr His Asp Thr Tyr Tyr Val Val Ala His Phe His Tyr Val Met Ser
                405                 410                 415
Leu Gly Ala Ile Phe Ala Ile Phe Ala Gly Ile Tyr Phe Tyr Met Pro
            420                 425                 430
Lys Phe Ser Gly Arg Ala Phe Pro Glu Trp Ala Ala Lys Leu His Phe
        435                 440                 445
Trp Thr Phe Phe Ile Gly Ala Asn Val Thr Phe Phe Pro Gln His Phe
    450                 455                 460
Leu Gly Arg Gln Gly Met Pro Arg Arg Tyr Ile Asp Tyr Pro Glu Ala
465                 470                 475                 480
Phe Ala Leu Trp Asn Lys Val Ser Ser Tyr Gly Ala Phe Leu Ala Phe
                485                 490                 495
Ala Ser Phe Leu Phe Phe Ile Val Ile Phe Val Tyr Thr Leu Val Ala
            500                 505                 510

Gly Arg Arg Glu Thr Arg Pro Asn Pro Trp Gly Glu Phe Ala Asp Thr

        515                 520                 525

Leu Glu Trp Thr Leu Pro Ser Pro Pro Pro Ala His Thr Phe Glu Thr

    530                 535                 540

Leu Pro Lys Arg Ser Asp Trp Asp Lys His Pro Ser His

545                 550                 555

<210> 3
<211> 132
<212> DNA
<213> Gluconobacter oxydans

<220>
<221> CDS
<222> (1)..(132)

<400> 3

ccg ctg gaa atc gtc tgg acg att gtt ccg gtt gtg att ctg gtc ttc 48

Pro Leu Glu Ile Val Trp Thr Ile Val Pro Val Val Ile Leu Val Phe

1               5                   10                  15

atc ggt gcg ttc tcg ctg ccg gtg ctg ttc aaa cag caa gag ttc ccc 96

Ile Gly Ala Phe Ser Leu Pro Val Leu Phe Lys Gln Gln Glu Phe Pro

            20                  25                  30

gag ggt gac atc gtc atc aac gtc gag ggt cgt agc 132

Glu Gly Asp Ile Val Ile Asn Val Glu Gly Arg Ser

        35                  40

<210> 4
<211> 44
<212> PRT
<213> Gluconobacter oxydans

<400> 4

Pro Leu Glu Ile Val Trp Thr Ile Val Pro Val Val Ile Leu Val Phe

1               5                   10                  15

Ile Gly Ala Phe Ser Leu Pro Val Leu Phe Lys Gln Gln Glu Phe Pro

20                  25                  30

Glu Gly Asp Ile Val Ile Asn Val Glu Gly Arg Ser

35                  40

<210> 5
<211> 114
<212> DNA
<213> Gluconobacter oxydans

<220>
<221> CDS
<222> (1)..(114)

<400> 5

atc gtc cac ggc gac cgc aag aaa acc gcg att ggc cta gcg att gcc   48

Ile Val His Gly Asp Arg Lys Lys Thr Ala Ile Gly Leu Ala Ile Ala

1               5                   10                  15

atc ggc ctt ggc tgg atc ttt acc ctg tgc caa gcc tat gaa tat tat   96

Ile Gly Leu Gly Trp Ile Phe Thr Leu Cys Gln Ala Tyr Glu Tyr Tyr

20                  25                  30

gaa atc gtc cat acc gaa                                           114

Glu Ile Val His Thr Glu

        35

<210> 6
<211> 38
<212> PRT

<213> Gluconobacter oxydans

<400> 6

Ile Val His Gly Asp Arg Lys Lys Thr Ala Ile Gly Leu Ala Ile Ala

1               5                   10                  15

Ile Gly Leu Gly Trp Ile Phe Thr Leu Cys Gln Ala Tyr Glu Tyr Tyr

            20                  25                  30

Glu Ile Val His Thr Glu

        35

<210> 7
<211> 87
<212> DNA
<213> Gluconobacter oxydans

<220>
<221> CDS
<222> (1)..(87)

<400> 7

gat tcg atc ttc ctg ctg gtc tgc ctg atc cgc atc ctg cgc ggt gcg   48

Asp Ser Ile Phe Leu Leu Val Cys Leu Ile Arg Ile Leu Arg Gly Ala

1               5                   10                  15

atg tcg gca aaa cag cac gtc ggt ttc gag atg gcc gca               87

Met Ser Ala Lys Gln His Val Gly Phe Glu Met Ala Ala

            20                  25

<210> 8
<211> 29
<212> PRT
<213> Gluconobacter oxydans

<400> 8

Asp Ser Ile Phe Leu Leu Val Cys Leu Ile Arg Ile Leu Arg Gly Ala

1               5                   10                  15

Met Ser Ala Lys Gln His Val Gly Phe Glu Met Ala Ala

            20                  25

<210> 9
<211> 6
<212> PRT
<213> Rhodobacter sphaeroides

<400> 9

Trp Phe Phe Gly His Pro

1               5

<210> 10
<211> 6
<212> PRT
<213> Rhodobacter sphaeroides

<400> 10

Val Trp Ala His His Met

1               5

<210> 11
<211> 16
<212> PRT
<213> Gluconobacter oxydans

<220>
<221> PEPTIDE
<222> (1)..(16)

<400> 11

Lys Asp Ile Gly Leu Leu Tyr Leu Val Ala Ala Gly Val Val Gly Phe

1               5                   10                  15

<210> 12
<211> 168
<212> DNA

<213> Gluconobacter oxydans

<220>
<221> CDS
<222> (1)..(168)

<400> 12

tgg ttt ttt gga cac ccg gaa gtg tac atc atc att ctg ccc ggc ttt   48

Trp Phe Phe Gly His Pro Glu Val Tyr Ile Ile Ile Leu Pro Gly Phe

1               5                   10                  15

ggc atc atc agc cat gtc gtg tcg acc ttc tcg aaa aag ccg gtc ttc   96

Gly Ile Ile Ser His Val Val Ser Thr Phe Ser Lys Lys Pro Val Phe

            20                  25                  30

ggt tac ctg ccg atg gtc tat gca atg ttg gca atc ggt gtt ctg ggc   144

Gly Tyr Leu Pro Met Val Tyr Ala Met Leu Ala Ile Gly Val Leu Gly

        35                  40                  45

ttt gtc gtg tgg gcg cac cat atg                                   168

Phe Val Val Trp Ala His His Met

    50                  55

<210> 13
<211> 56
<212> PRT
<213> Gluconobacter oxydans

<400> 13

Trp Phe Phe Gly His Pro Glu Val Tyr Ile Ile Ile Leu Pro Gly Phe

1               5                   10                  15

Gly Ile Ile Ser His Val Val Ser Thr Phe Ser Lys Lys Pro Val Phe

            20                  25                  30

Gly Tyr Leu Pro Met Val Tyr Ala Met Leu Ala Ile Gly Val Leu Gly

        35                  40                  45

Phe Val Val Trp Ala His His Met

    50                  55

<210> 14
<211> 20
<212> PRT
<213> Gluconobacter oxydans

<220>
<221> PEPTIDE
<222> (1)..(20)

<400> 14

Lys Ala Ser Gln Phe Thr His Asn Thr Pro Leu Glu Ile Val Trp Thr

1               5                   10                  15

Ile Val Pro Val

            20

<210> 15
<211> 6
<212> PRT
<213> Gluconobacter oxydans

<400> 15

Gln Phe Thr His Asn Thr

1               5

<210> 16
<211> 7
<212> PRT
<213> Rhodobacter sphaeroides

<400> 16

Trp Tyr Trp Gly Tyr Glu Tyr

1               5

<210> 17
<211> 6
<212> PRT
<213> Rhodobacter sphaeroides

<400> 17

Thr Trp Ala His His Ala

1               5

<210> 18
<211> 7
<212> PRT
<213> Rhodobacter sphaeroides

<400> 18

Trp Tyr Trp His Phe Val Asp

1               5